(19) Europäisches Patentamt / European Patent Office / Office européen des brevets

(11) **EP 3 942 300 B1**

(12) **FASCICULE DE BREVET EUROPEEN**

(45) Date de publication et mention de la délivrance du brevet:
**19.08.2026 Bulletin 2026/34**

(21) Numéro de dépôt: **20728113.0**

(22) Date de dépôt: **18.03.2020**

(51) Classification Internationale des Brevets (IPC):
***G01N 33/543*** *(2006.01)* ***C07D 213/38*** *(2006.01)*
***C07D 401/14*** *(2006.01)* ***G01N 33/68*** *(2006.01)*
***C07F 1/08*** *(2006.01)* ***C07F 3/06*** *(2006.01)*

(52) Classification Coopérative des Brevets (CPC):
**G01N 33/54353; C07F 1/08; C07F 3/06; G01N 33/6893; G01N 33/6896**

(86) Numéro de dépôt international:
**PCT/FR2020/050593**

(87) Numéro de publication internationale:
**WO 2020/188223 (24.09.2020 Gazette 2020/39)**

(54) **CAPTURE DE MICROVESICULES A VISEE DIAGNOSTIQUE**

AUFNAHME VON MIKROBLÄSCHEN FÜR DIAGNOSTISCHE ZWECKE

CAPTURE OF MICROVESICLES FOR DIAGNOSTIC PURPOSES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.03.2019 FR 1902814**

(43) Date de publication de la demande:
**26.01.2022 Bulletin 2022/04**

(73) Titulaires:
- **UNIVERSITE DE BORDEAUX 33000 Bordeaux (FR)**
- **Institut Polytechnique de Bordeaux 33400 Talence (FR)**
- **Centre national de la recherche scientifique 75016 Paris (FR)**
- **Centre Hospitalier Universitaire de Bordeaux 33404 Talence Cedex (FR)**

(72) Inventeurs:
- **PLAWINSKI, Laurent 33830 BELIN BELIET (FR)**
- **DURRIEU, Marie-Christine 33000 BORDEAUX (FR)**
- **PETITET, Marion 33400 TALENCE (FR)**
- **LAVIE, Julie 33140 VILLENAVE D'ORNON (FR)**
- **OERTHEL, Marie Christine 33140 VILLENAVE D'ORNON (FR)**
- **NLATE, Sylvain 33600 PESSAC (FR)**
- **CHANSEAU, Christel 33000 BORDEAUX (FR)**
- **RIGALLEAU, Vincent 33600 PESSAC (FR)**
- **ALEXANDRE, Laure 33000 BORDEAUX (FR)**

(74) Mandataire: **Santarelli Tour Trinity 1 bis Place de la Défense 92400 Courbevoie (FR)**

(56) Documents cités:
**WO-A1-2012/127175**
**CN-A- 103 333 878**
**CN-A- 107 754 014**
**ES-A1- 2 340 131**
**ES-B1- 2 340 131**

EP 3 942 300 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Processed by Luminess, 75001 PARIS (FR)

**(Cont. page suivante)**

- WANG HAO ET AL: "An effective non-covalent grafting approach to functionalize individually dispersed reduced graphene oxide sheets with high grafting density, solubility and electrical conductivity", NANOSCALE, vol. 7, no. 8, 13 November 2015 (2015-11-13), United Kingdom, pages 3548 - 3557, XP093027492, ISSN: 2040-3364, Retrieved from the Internet <URL:https://pubs.rsc.org/en/content/articlepdf/2015/nr/c4nr06710j> DOI: 10.1039/C4NR06710J
- HARA M ET AL: "Urinary podocalyxin is an early marker for podocyte injury in patients with diabetes: establishment of a highly sensitive ELISA to detect urinary podocalyxin", DIABETOLOGIA ; CLINICAL AND EXPERIMENTAL DIABETES AND METABOLISM, SPRINGER, BERLIN, DE, vol. 55, no. 11, 2 August 2012 (2012-08-02), pages 2913 - 2919, XP035121284, ISSN: 1432-0428, DOI: 10.1007/S00125-012-2661-7
- ANNE STUENDL ET AL: "Induction of [alpha]-synuclein aggregate formation by CSF exosomes from patients with Parkinson's disease and dementia with Lewy bodies", BRAIN, vol. 139, no. 2, 8 December 2015 (2015-12-08), pages 481 - 494, XP055314034, ISSN: 0006-8950, DOI: 10.1093/brain/awv346
- KATHRYNA FONTANA RODRIGUES ET AL: "Circulating microparticles are associated with type 2 diabetes mellitus and correlate with fasting glucose levels", JOURNAL OF DIABETOLOGY AND METABOLIC SYNDROME, vol. 10(Supp 1), 1 January 2018 (2018-01-01), pages A60, XP055641757
- MILLER D W ET AL: "Unaltered @a-synuclein blood levels in juvenile Parkinsonism with a parkin exon 4 deletion", NEUROSCIENCE LETTERS, ELSEVIER, AMSTERDAM, NL, vol. 374, no. 3, 21 February 2005 (2005-02-21), pages 189 - 191, XP004714811, ISSN: 0304-3940, DOI: 10.1016/J.NEULET.2004.10.053
- A. J. STEELE ET AL: "The JAK3-selective inhibitor PF-956980 reverses the resistance to cytotoxic agents induced by interleukin-4 treatment of chronic lymphocytic leukemia cells: potential for reversal of cytoprotection by the microenvironment", BLOOD, vol. 116, no. 22, 17 August 2010 (2010-08-17), US, pages 4569 - 4577, XP055573311, ISSN: 0006-4971, DOI: 10.1182/blood-2009-09-245811

## Description

La présente invention concerne des supports fonctionnalisés et leur utilisation pour le diagnostic de pathologies.

ARRIERE-PLAN TECHNOLOGIQUE

Les microvésicules cellulaires sont des vésicules émises dans le milieu extracellulaire *via* le bourgeonnement d'une cellule activée suite à un stress. Les microvésicules larguées dans les fluides biologiques (sang, urine, larme, etc.) renferment et/ou portent à leur surface des constituants de la cellule-mère (lipides, protéines et ARN, ou ADN mitochondrial) et donc peuvent être considérées comme des marqueurs précoces d'un état pathologique d'un tissu.

Une méthode décrite dans la demande internationale PCT/FR2012/050610 emploie un ligand synthétique pour capter des microvésicules phosphatidylsérine-positive. Un tel ligand synthétique est lié à un support solide par intermédiaire d'une couche de polyglutaraldéhyde qui est préalablement déposé par adsorption sur le support solide.

La demande de brevet ES 2 340 131 décrit un procédé d'obtention de polystyrène modifié avec des groupes chlorosulfonyles.

La demande de brevet CN 103 333 878 décrit un procédé de préparation d'une protéase à haute activité immobilisée sur un support de chlorure de polyvinyle modifié.

La demande de brevet CN 107 754 014 décrit un procédé de préparation d'un revêtement composite antibactérien favorisant la croissance osseuse sur une surface de fibre de polytéréphtalate d'éthylène (PET). Ce procédé consiste à utiliser du chitosane, un polysaccharide alcalin naturel, et de l'hydroxyapatite (HAp), un sel inorganique semblable à l'os, comme matériaux de revêtement.

Hara et al. (Diabetologia, 2012, vol. 55 :2913-2919) décrivent la podocalyxine urinaire comme un marqueur précoce de lésion des podocytes chez les patients diabétiques.

Stuendl et al. (Brain, 2015, vol. 139, no. 2, p. 481-494) décrivent les résultats expérimentaux obtenus dans des exosomes isolés à partir du liquide cérébrospinal de patients atteints de la maladie de Parkinson et de démence à corps de Lewy. Les exosomes ont été isolés par des cycles de centrifugation.

Rodrigues et al. (Journal of Diabetology & Metabolic Syndrome, 2018, vol. 10 (supp 1) : A60) décrivent le lien entre le niveau des microparticules circulantes et le diabète de type 2 (T2DM).

Miller et al. (Neuroscience Letters, 2005, vol. 374, p. 189-191) décrivent qu'il n'y a pas d'augmentation de l'$\alpha$-synucléine dans le parkinsonisme juvénile autosomique récessif.

Steele et al. (Blood. 2010, vol 116(22), p. 4569-4577) décrivent que l'inhibiteur sélectif de JAK3, PF-956980, est capable d'inverser la résistance aux agents cytotoxiques induite par le traitement à l'interleukine-4 dans des cellules de la leucémie lymphoïde chromique.

Wang et al. (Nanoscale, 2015, vo ; 7, 3548) décrivent une méthode de greffage non covalent pour fonctionnaliser des feuillets d'oxyde de graphène réduit dispersés individuellement.

La présente invention concerne un perfectionnement des moyens et méthodes de capture présentés dans la demande PCT/FR2012/050610. Elle concerne également l'identification de biomarqueurs de pathologies d'intérêt au moyen de supports fonctionnalisés selon les modalités présentées ci-dessous.

RESUME DE L'INVENTION

L'invention est définie dans les revendications.

L'invention concerne un support solide fonctionnalisé, utile pour la capture, la détection et/ou la caractérisation de microvésicules cellulaires. Le support selon l'invention est également utile dans le domaine médical pour la mise en œuvre de méthodes de diagnostic et/ou de pronostic, notamment pour le diagnostic précoce d'une maladie.

Le support selon l'invention est un support solide, notamment polymérique, métallique ou céramique, comprenant un composé de formule (I) ou (II) tel que décrit ci-dessous qui est lié de manière covalente au support de manière directe ou indirecte, dans lequel, dans le cas d'une liaison covalente indirecte, ledit composé de formula (I) ou (II) est lié de manière covalente à une fonction réactive apportée à la surface du support par un agent de pré-fonctionnalisation, qui a également été lié de manière covalente au support. Selon un mode particulier de réalisation, le support est un support polymérique, notamment un support en poly(chlorure de vinyle) (ou PVC), en poly(téréphtalate d'éthylène) (ou PET), ou en polystyrène (ou PS), fonctionnalisé au moyen d'un composé de formule (I) ou (II) tel que décrit ci-dessous.

L'invention concerne également une méthode pour le diagnostic d'une pathologie, comprenant la détection de la présence ou de l'absence de marqueurs spécifiques identifiés par les inventeurs. Cette détection peut notamment être réalisée après capture de microvésicules selon les modalités présentées dans la présente demande.

LEGENDE DES FIGURES

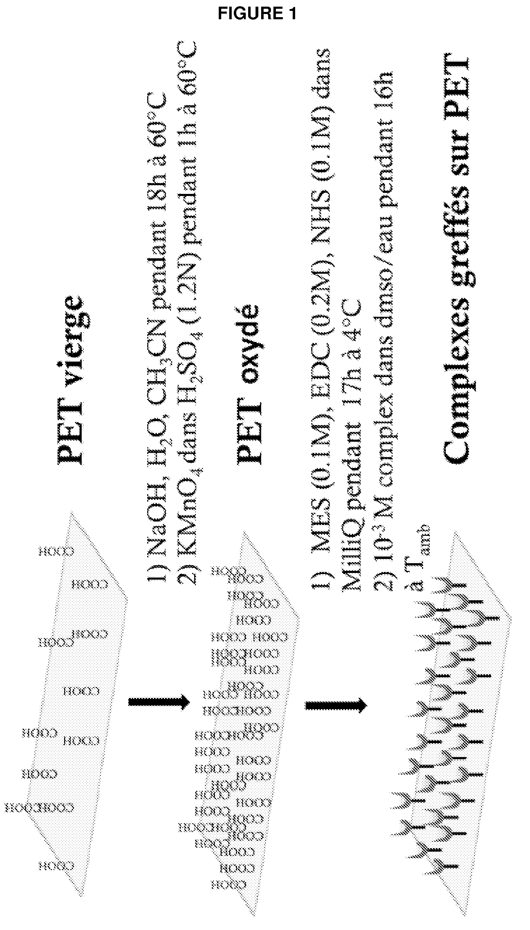

La figure 1 représente des étapes de fonctionnalisation du PET.

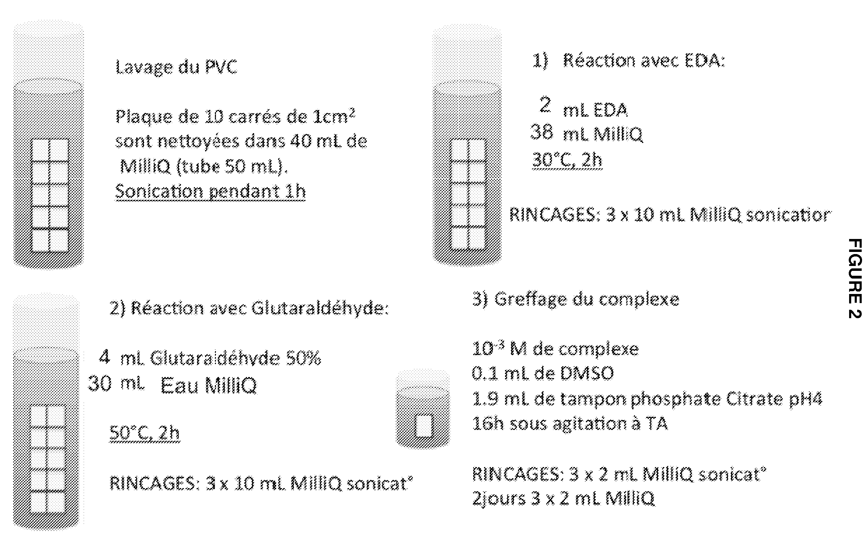

La figure 2 représente des étapes de fonctionnalisation du PVC.

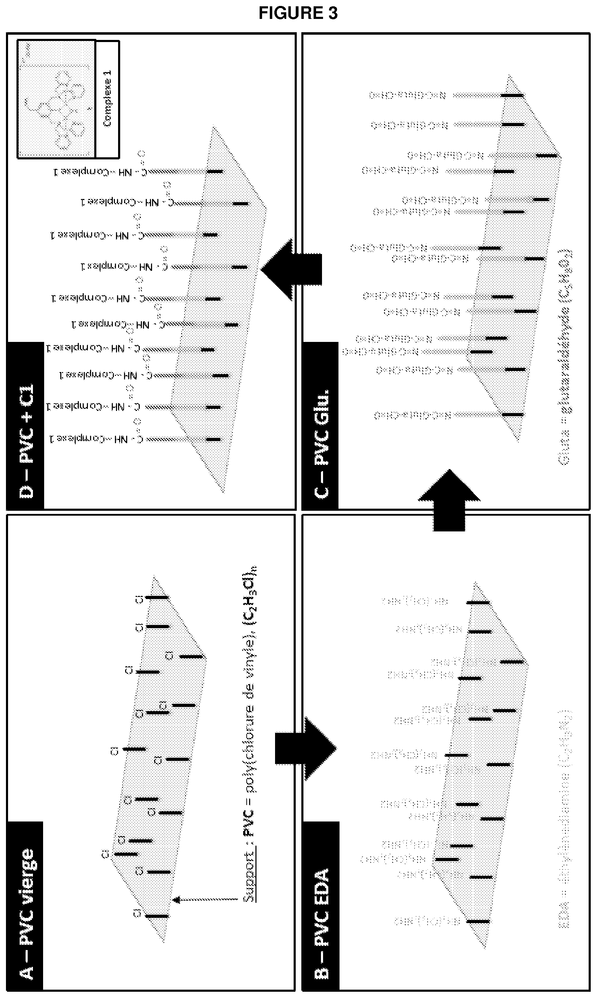

La figure 3 représente une schématisation de la surface du PVC à chaque étape de sa fonctionnalisation avec le complexe 1. (A) Etape 1 : PVC vierge. (B) Etape 2 : ajout de l'éthylènediamine (EDA). (C) Etape 3 : ajout du glutaraldéhyde (Glu.). (D) Etape 4 : ajout du complexe 1 (C1).

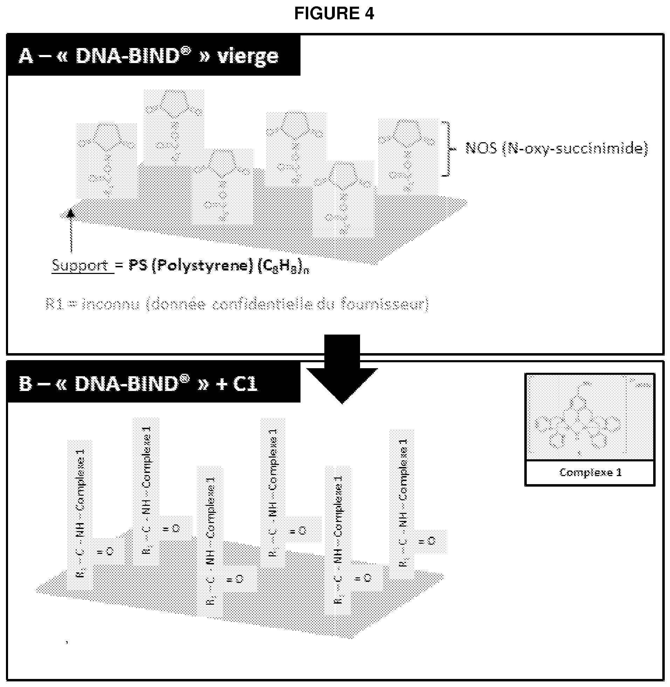

La figure 4 représente une schématisation de la surface du « DNA-BIND® » à chaque étape de sa fonctionnalisation avec le complexe 1. (A) Etape 1 : «DNA-BIND®» vierge. (B) Etape 2 : ajout du complexe 1 (C1).

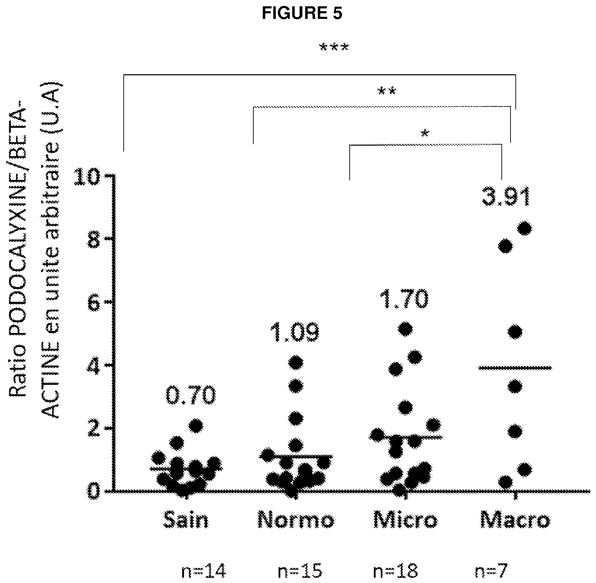

La figure 5 est une représentation graphique du ratio podocalyxine/beta-actine dans les microvésicules issues d'urines de donneurs sains et de patients diabétiques. Chaque point représente le ratio obtenu pour un patient. La valeur de la moyenne mathématique est indiquée au-dessus de chaque groupe de patients. Les statistiques ont été analysés avec un test de Tukey's ANOVA. * : $p<0.05$ ; ** : $p<0.01$ et *** : $p<0.001$. Légendes : n = nombre de patients, Sain : Donneurs sains ; Normo : Patients Normoalbuminuriques, Micro : Patients Microabulminuriques ; Macro : Patients Macroalbuminuriques.

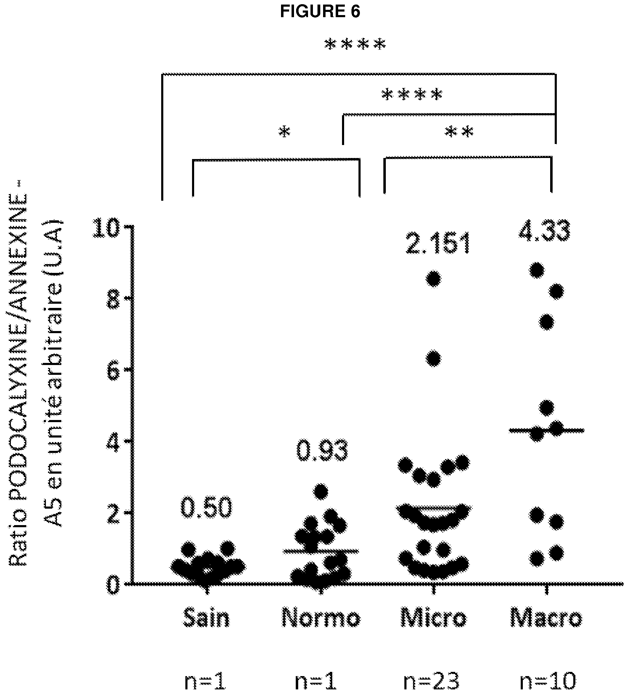

La figure 6 est une représentation graphique du ratio podocalyxine/annexine-A5 dans les microvésicules issues d'urines de donneurs sains et de patients diabétiques. Chaque point représente le ratio obtenu pour un patient. La valeur de la moyenne mathématique est indiquée au-dessus de chaque groupe de patients. Les statistiques ont été analysés avec un test de Tukey's ANOVA. * : $p<0.05$ ; ** : $p<0.01$ ; *** : $p<0.001$ et **** : $p<0.0001$. Légendes : n = nombre de patients, Sain : Donneurs sains ; Normo : Patients Normoalbuminuriques, Micro : Patients Microabulminuriques ; Macro : Patients Macroalbuminuriques.

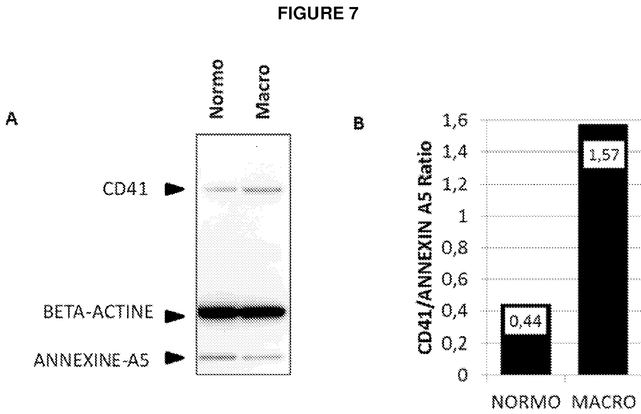

La figure 7 représente la détection du marqueur plaquettaire CD41 et représentation graphique du ratio CD41/annexine-A5 dans les microvésicules issues de plasma de patients diabétiques. A. Electrophorèse des protéines de microvésicules issues de plasma de deux patients diabétiques ; un normoalbuminurique (Normo) et un macroalbuminurique (Macro). Révélation du western blot à l'aide d'anticorps contre le CD41, et les protéines de référence beta-actine et annexine-A5. B. Quantification du signal des bandes relatives à CD41, et annexine-A5 à l'aide du logiciel ImageJ et représentation graphique du ratio CD41/annexine-A5.

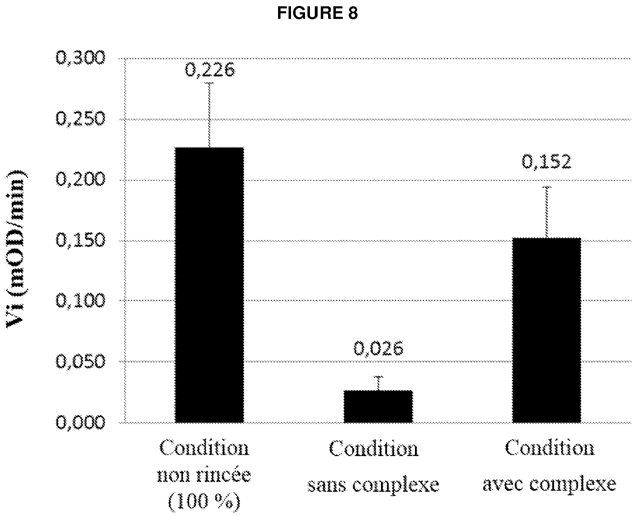

La figure 8 représente la détection de l'activité enzymatique de l'uPA dans les microvésicules isolées à partir de cellules en culture HUVECs. Représentation graphique des moyennes des vitesses initiales mesurées en mOD/min par spectrophotométrie. Légende : Condition non rincée contrôle=100% ; condition rincée sans complexe=sans matériau ; condition rincée avec complexe=avec matériau.

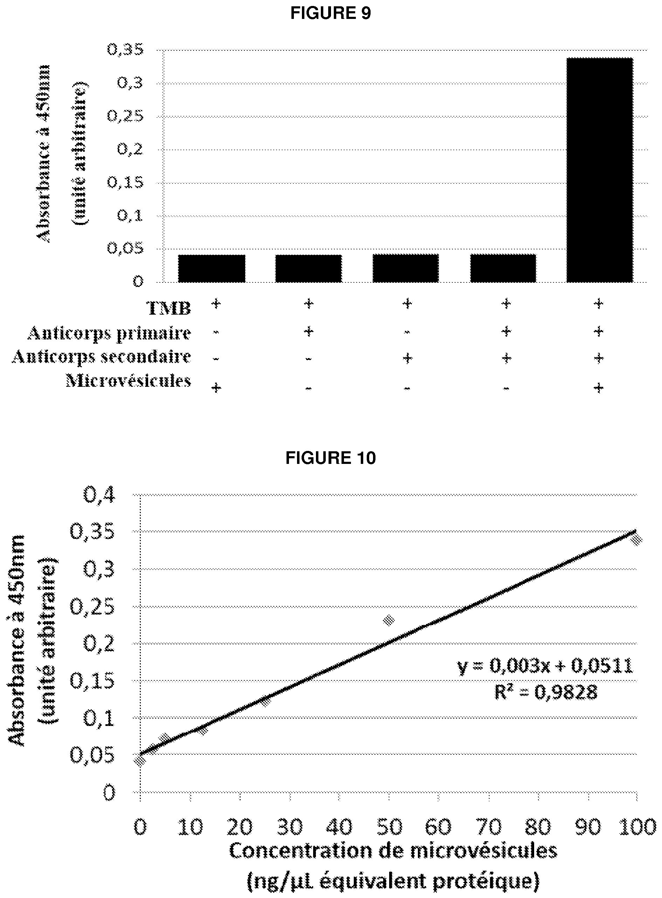

La figure 9 représente la détection immunologique de la podocalyxine dans les microvésicules isolées à partir d'urine humaine et captées par le matériau. Représentation graphique des absorbances obtenues par spectrophotométrie à 450 nm dans les différentes conditions : 1-Microvésicules + TMB, 2- Anticorps primaire + TMB, 3- Anticorps secondaire + TMB, 4- Anticorps primaire + Anticorps secondaire + TMB, 5- Anticorps primaire + Anticorps secondaire + TMB.

La figure 10 représente la détection immunologique de la podocalyxine en fonction de la concentration de microvésicules d'urine humaine captées par le matériau. Représentation graphique des absorbances obtenues par spectrophotométrie à 450 nm en fonction de la concentration des microvésicules exprimée en ng/$\mu$L. L'équation de la droite et le $R^2$ sont calculés par le logiciel Excel.

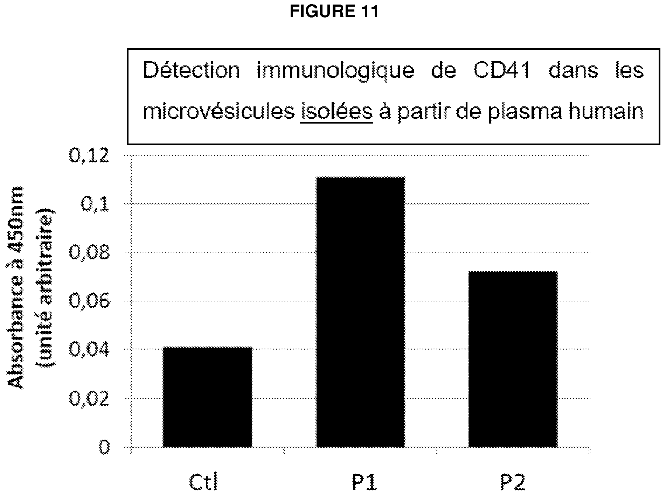

La figure 11 représente la détection immunologique de CD41 dans les microvésicules isolées à partir de plasma humain et captées par le matériau. Représentation graphique des signaux détectés pour la présence de CD41 dans les microvésicules des patients diabétiques normoalbuminuriques P1 et P2. La condition contrôle (Ctl) représente le bruit de fond enregistré dans le puits sans microvésicules en présence du réactif TMB.

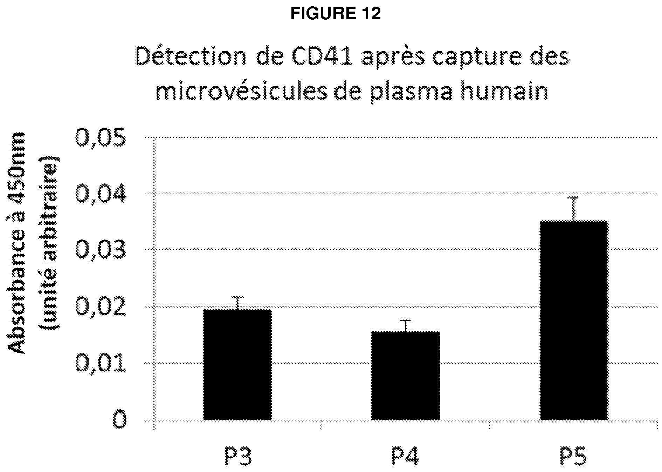

La figure 12 représente la détection immunologique de CD41 dans le plasma humain après captures des microvésicules par le matériau. Représentation graphique des signaux détectés pour la présence de CD41 dans les microvésicules des patients diabétiques normoalbuminuriques P3, P4 et P5. Les signaux sont calculés après déduction du signal obtenu pour le bruit de fond (c'est à dire en présence de tous les réactifs mais sans anticorps primaire).

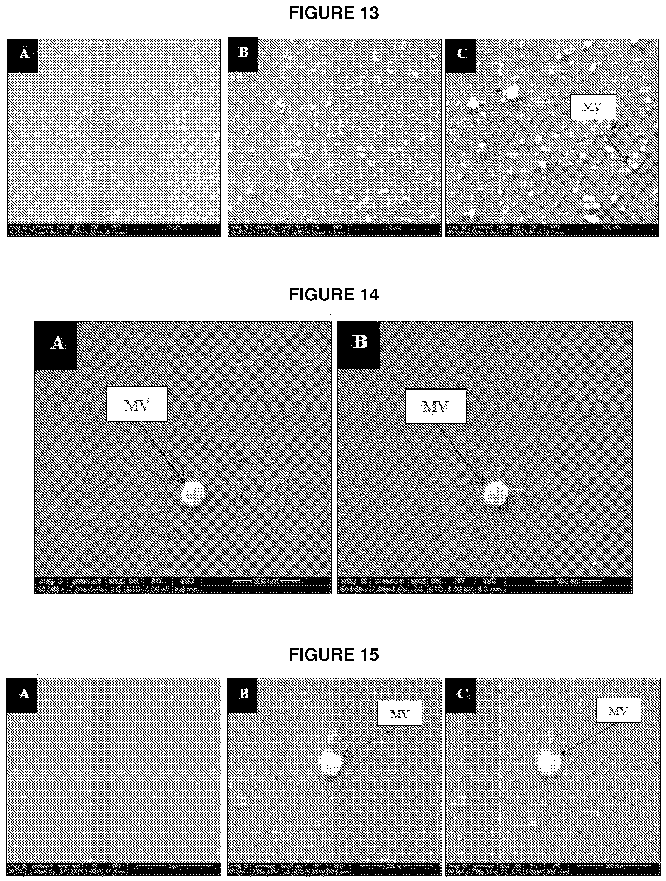

La figure 13 représente une analyse CryoMEB de la capture des microvésicules issues de l'activation cellulaire des cellules HUVEC, par le matériau PVC + C1. (A) Barre d'échelle 10 $\mu$m. (B) Barre d'échelle 2 $\mu$m. (C) Barre d'échelle 500 nm.

La figure 14 représente une analyse CryoMEB de la capture des microvésicules, issues de l'activation cellulaire des cellules HUVEC, par le matériau « DNA-BIND®» + C1. (A) Barre d'échelle 500 nm. (B) Barre d'échelle 500 nm et mesure du diamètre d'une microvésicule.

La figure 15 représente une analyse CryoMEB de la capture des microvésicules, issues de prélèvement urinaire

humain de sujet sain, par le matériau « DNA-BIND®» + C1. (A) Barre d'échelle 5 μm. (B) Barre d'échelle 500 nm. (C) Barre d'échelle 500 nm et mesure du diamètre d'une microvésicule.

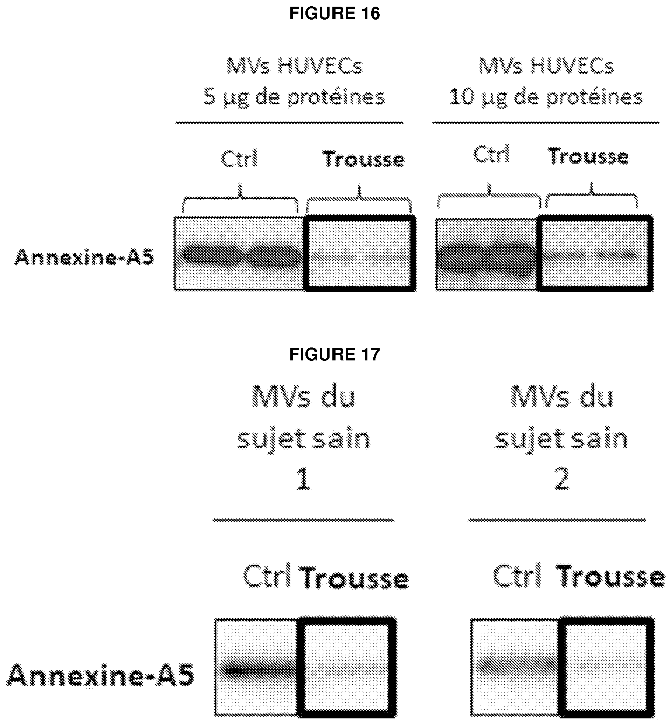

La figure 16 représente la détection du marqueur annexine-A5 sur microvésicules de modèle cellulaire HUVEC à deux doses (5 μg et 10 μg), en duplicat, sans capture (Contrôle, Ctrl) et après 1h de capture sur la trousse à 37°C (Trousse).

La figure 17 représente la détection du marqueur annexine-A5 sur microvésicules de prélèvement urinaire de deux sujets sains sans capture (Contrôle, Ctrl) et après 1 nuit de capture par la trousse à 4°C (Trousse).

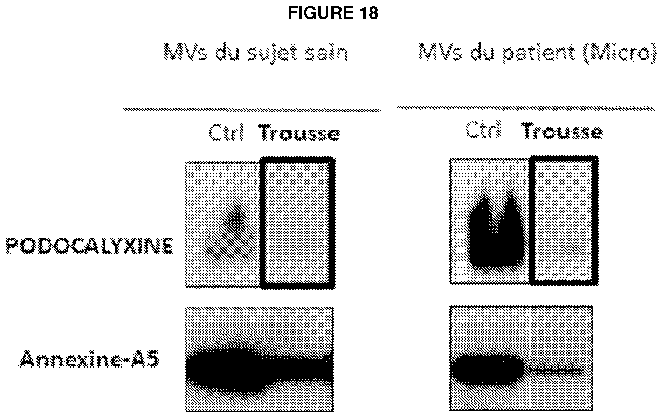

La figure 18 représente la détection du biomarqueur pathologique podocalyxine et du marqueur de référence microvésiculaire annexine-A5 sur les microvésicules de prélèvements urinaires d'un sujet sain et d'un patient diabétique atteint de néphropathie au stade microalbuminurique (Micro) sans capture (Contrôle, Ctrl) et après 1 nuit de capture par la trousse à 4°C (Trousse).

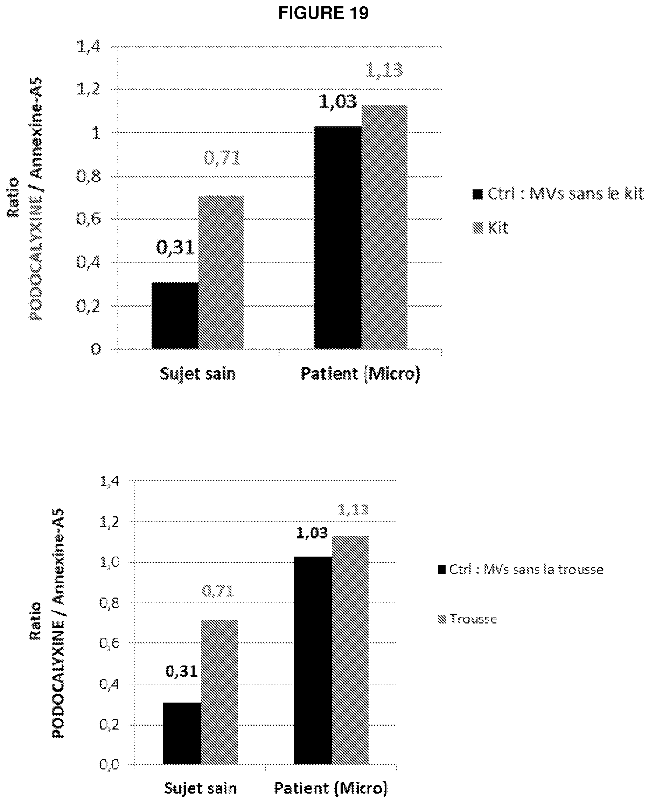

La figure 19 représente la comparaison du ratio podocalyxine/annexine-A5 correspondant aux microvésicules de prélèvements urinaires d'un sujet sain et d'un patient diabétique atteint de néphropathie au stade microalbuminurique (Micro) sans capture (Contrôle, Ctrl) et après 1 nuit de capture par la trousse à 4°C (Trousse).

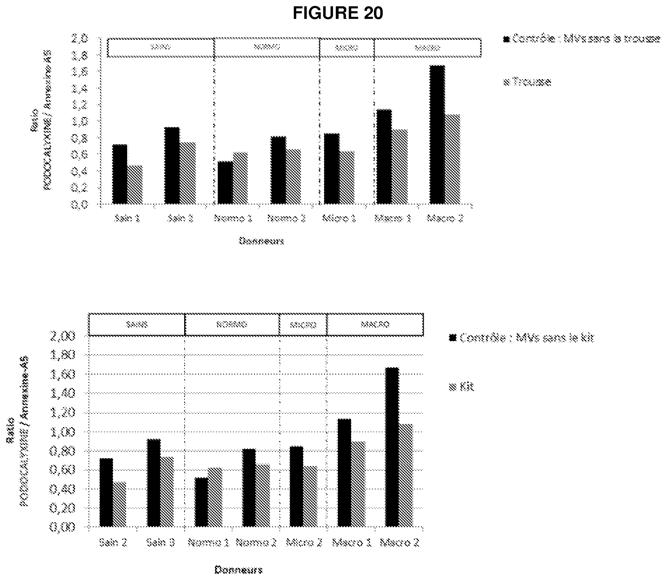

La figure 20 représente la comparaison du ratio podocalyxine/annexine-A5 correspondant aux microvésicules des prélèvements urinaires de sujets sains et de patients diabétiques atteints de néphropathie au stade Normo, Micro et Macro sans capture (Contrôle, Ctrl) et après 1 nuit de capture par la trousse à 4°C (Trousse).

DESCRIPTION DETAILLEE DE L'INVENTION

Selon un premier aspect, l'invention concerne un support solide caractérisé en ce qu'il comprend un composé de formule (I) ou (II) suivante qui est lié de manière covalente au support de manière directe ou indirecte :

(I)

(II)

dans laquelle

- $M^{+i}$ représente un ion métallique et i est 1, 2 ou 3;
- L représente un ligand échangeable;
- X représente un groupe $-(CH_2)_m-NH_2$, ou un groupe $-CH_2-NHC(O)-R-NH_2$ dans lequel R est un groupe alkyle en $C_2-C_{10}$, en particulier en $C_5-C_{10}$, substitué ou non substitué, linéaire ou ramifié;
- m = 1 à 12;
- n = 1, 2 ou 3; et
- Y représente H ou $(CH_2)_p-NH_2$, où p = 0 à 12.

dans lequel, dans le cas d'une liaison covalente indirecte, ledit composé de formula (I) ou (II) est lié de manière covalente à une fonction réactive apportée à la surface du support par un agent de pré-fonctionnalisation, qui a également été lié de manière covalente au support.

Selon la présente invention, le composé de formule (I) ou (II) est greffé de manière covalente sur le support solide. Le procédé décrit dans la demande PCT/FR2012/050610 n'enseigne pas un greffage covalent des complexes à la surface des supports. Y est plutôt décrite une adsorption non covalente de polyglutaraldéhyde sur un support puis une immobilisation du complexe sur le polyglutaraldéhyde via des fonctions $NH_2$ dudit complexe. Avantageusement, grâce à la méthode de la présente invention, l'accessibilité du complexe pour la phosphatidylserine est plus aisée. Par ailleurs, la méthode selon la présente invention permet d'avoir une meilleure reproductibilité de la fonctionnalisation. La méthode selon la présente invention permet ainsi un contrôle précis de la densité de complexes immobilisés de manière covalente, et a notamment pour avantages:

- un meilleur contrôle de la présentation du complexe pour une meilleure interaction avec la phosphatidylserine présente dans les microvésicules;
- un meilleur contrôle de la densité de greffage du complexe;
- aucune désorption possible dans le milieu;
- une meilleure reproductibilité de la fonctionnalisation d'un matériau à l'autre d'un lot à l'autre.

Les composés de formule (I) ou (II) sont cationiques, leur contre-ion pouvant être choisi, par exemple, parmi les anions tosylate, nitrate, sulfate, sulfonate, thiosulfate, halogénure, hexafluorophosphate, tétraphénylborate, tétrafluoroborate, perchlorate, etc, notamment les anions perchlorate, nitrate, sulfate, halogénure et carbonate.

Selon un mode particulier de réalisation, M est choisi parmi Zn, Cu, Mn, Co, Ni et Fe, Zn ou Cu étant préférés, plus particulièrement Zn.

Selon un mode particulier de réalisation, Y représente H.

Selon d'autres modes particuliers de réalisation :

- X représente $-(CH_2)_m-NH_2$, m= 1, n=1 et Y représente H; ou
- X représente $-(CH_2)_m-NH_2$, m= 1, n=2 et Y représente H; ou
- X représente $-CH_2-NHC(O)-R-NH_2$, R représente C5H10, n=1 ou 2 et Y représente H.

Selon un mode particulier de réalisation, le composé lié de manière covalente au support de manière directe ou indirect est un composé de formule (I).

Selon un autre mode particulier de réalisation, le composé de formule (I) est choisi parmi:

· $(ClO_4)_2$

(Ia);

· $(ClO_4)_2$

(Ib);

· $(ClO_4)_2$

(Ic); et

(Id).

Il est entendu que la présente demande décrit également les composés de formule (Ia), (Ib), (Ic) et (Id) associés à un contre-ion différent du perchlorate, notamment choisi parmi les anions tosylate, nitrate, sulfate, sulfonate, thiosulfate, halogénure, hexafluorophosphate, tétraphénylborate et tétrafluoroborate, plus particulièrement parmi les anions nitrate, sulfate, halogénure et carbonate. Par ailleurs, le sel utilisé pour générer le contre-ion peut notamment être un sel de zinc, un sel de cuivre, un sel de manganèse, un sel de cobalt, un sel de nickel ou encore un sel de fer, plus particulièrement un sel de zinc, notamment le perchlorate de zinc, le nitrate de zinc, le sulfate de zinc, un halogénure de zinc ou un carbonate de zinc.

Selon un mode particulier de réalisation, le composé est un composé de formule:

(Ia);

étant entendu que le contre-ion pouvant être différent du perchlorate, et notamment choisi parmi les anions tosylate, nitrate, sulfate, sulfonate, thiosulfate, halogénure, hexafluorophosphate, tétraphénylborate et tétrafluoroborate, plus particulièrement parmi les anions nitrate, sulfate, halogénure et carbonate. Selon un mode particulier de réalisation, le contre-ion est un anion perchlorate. Par ailleurs, le sel utilisé pour générer le contre-ion peut notamment être un sel de zinc, un sel de cuivre, un sel de manganèse, un sel de cobalt, un sel de nickel ou encore un sel de fer, plus particulièrement un sel de zinc, notamment le perchlorate de zinc, le nitrate de zinc, le sulfate de zinc, un halogénure de zinc ou un carbonate de zinc.

Les composés de formule (I) ou (II) peuvent être préparés selon les modalités présentées dans la demande PCT/FR2012/050610.

Le support solide employé dans le cadre de l'invention peut notamment être une plaque de microtitration, une feuille, un cône, un tube, un puits, une bille, une particule, une bandelette, un film, un fil, une vis ou une aiguille. De manière générale, le support solide selon l'invention peut être employé pour la fabrication de tout type de matériau à géométrie et

porosité variable.

Dans un mode particulier de réalisation, le support solide est un support polymérique, métallique ou céramique, fonctionnalisé au moyen d'un composé de formule (I) ou (II). Selon un mode particulier de réalisation, le support est un support polymérique, notamment un support en poly(chlorure de vinyle) (ou PVC), en poly(téréphtalate d'éthylène) (ou PET), ou en polystyrène (ou PS).

Dans le contexte de l'invention, on entend par "greffé à sa surface", en référence au greffage du composé de formule (I) ou (II) sur le support, une liaison covalente entre le support et le composé de formule (I) ou (II). Comme décrit ci-dessous, le composé de formule (I) ou (II) peut être lié de manière covalente au support de manière directe ou indirecte. Dans le cas d'une liaison covalente indirecte, le composé de formula (I) ou (II) est lié de manière covalente à une fonction réactive apportée à la surface du support par un agent de pré-fonctionnalisation, qui a également été lié de manière covalente au support.

Le support solide peut être prétraité avant greffage du composé de formule (I) ou (II). Le prétraitement peut notamment avoir pour but de rompre des fonctions indésirables à la surface du support, notamment des fonctions esters, d'augmenter la densité de fonctions souhaitées, et/ou de pré-fonctionnaliser le support en le rendant plus électrophile. Dans le contexte de la présente invention, lorsque le support est pré-fonctionnalisé, ladite pré-fonctionnalisation est réalisée de manière covalente au moyen d'un agent de pré-fonctionnalisation. Selon un mode particulier de réalisation, le support utilisé est pré-fonctionnalisé. Le support peut ainsi être préfonctionnalisé au moyen de glutaraldéhyde, de N-hydroxysuccinimide (NHS) ou de N-oxysuccinimide (NOS) par exemple. Selon un mode de réalisation représentatif dans lequel le support est notamment en PET, le prétraitement peut notamment comprendre l'hydrolyse des fonctions esters présentes à la surface du support, l'augmentation de la densité en fonctions COOH, notamment par oxydation du support, en particulier au moyen de permanganate de potassium, et l'augmentation du caractère électrophile de cette surface par modification avec un groupement attracteur et bon groupe partant comme le groupement NHS. Selon un mode particulier de réalisation, le PET utilisé (PET Oxydé) comprend une densité de fonctions COOH à sa surface comprise entre 1 x $10^{12}$ COOH/$cm^2$ et 1 x $10^{18}$ COOH/$cm^2$, en particulier entre 1 x $10^{13}$ et 1 x $10^{17}$, plus particulièrement entre 1,2 x $10^{15}$ et 1,2 x $10^{17}$, telle que mesurée au moyen de bleu de toluidine oxydé (TBO). Selon une variante, le support est en PET et comprend entre 5 x $10^{15}$ et 5 x $10^{16}$ de fonctions COOH/$cm^2$ à sa surface avant pré-fonctionnalisation, plus particulièrement 1,26 x $10^{16}$ $\pm$ 10% de fonctions COOH/$cm^2$, notamment avant sa pré-fonctionnalisation au moyen d'un groupement NHS.

Ainsi, selon un mode de réalisation, le support est un support en PET, notamment en PET pré-fonctionnalisé, en particulier au moyen de fonctions NHS. Selon un mode particulier de réalisation, le PET pré-fonctionnalisé comprend une densité de fonctions COOH à sa surface comprise entre 1 x $10^{13}$ COOH/$cm^2$ et 1 x $10^{18}$ COOH/$cm^2$, en particulier entre 1 x $10^{14}$ et 1 x $10^{17}$, plus particulièrement entre 8,1 x $10^{14}$ et 8,2 x $10^{16}$. Selon une variante, le support en PET pré-fonctionnalisé comprend entre 5 x $10^{15}$ et 1 x $10^{16}$ de fonctions COOH/$cm^2$ à sa surface, plus particulièrement 8,18 x $10^{15}$ $\pm$ 10% de fonctions COOH/$cm^2$.

Selon un autre mode particulier de réalisation, le support est en PVC, notamment en PVC pré-fonctionnalisé, en particulier au moyen de glutaraldéhyde.

Selon un mode particulier de réalisation, le support solide est un support en PS, notamment en PS pré-fonctionnalisé, en particulier au moyen de fonctions N-oxysuccinimide (NOS). En particulier, la densité en fonctions NOS du support en PS ainsi pré-fonctionnalisé peut être comprise entre $10^{13}$ et $10^{16}$ NOS/$cm^2$, notamment entre $10^{14}$ et $10^{15}$ NOS/$cm^2$, la densité étant plus particulièrement d'environ 68 x $10^{14}$ NOS/$cm^2$.

Le composé de formule (I) ou (II) est immobilisé de manière covalente sur le support solide, éventuellement pré-fonctionnalisé, par introduction dudit support dans une solution comprenant le composé de formule (I) ou (II) à greffer. Selon le mode de réalisation où le support est pré-fonctionnalisé, l'agent de pré-fonctionnalisation est lié de manière covalente au support. Selon un mode particulier de réalisation, la solution comprend entre $10^{-5}$ et $10^{-2}$ M de composé de formule (I) ou (II), notamment entre $10^{-4}$ et 5 x $10^{-2}$ M, plus particulièrement entre 5 x $10^{-4}$ et 5 x $10^{-3}$ M. Le composé de formule (I) ou (II) peut notamment être à une concentration d'environ $10^{-3}$ M dans la solution. Le temps d'immobilisation peut varier dans une large mesure. Cependant, selon un mode particulier de réalisation, le support est mis en contact avec le composé de formule (I) ou (II) entre 1 h et 72 h, en particulier entre 5 h et 48 h, plus particulièrement entre 10 h et 24 h, et encore plus particulièrement pendant environ 16 h.

L'invention concerne par ailleurs un support selon l'invention, ledit support étant inclus dans une trousse destinée à la détection ou la caractérisation de microvésicules cellulaires. Une telle trousse peut avantageusement être employée, notamment en laboratoire d'analyse médicale, pour le diagnostic de pathologies d'intérêt. La trousse selon l'invention comprend un support selon l'invention, et peut éventuellement tout moyen susceptible d'être utilisé pour la mise en œuvre de la méthode selon l'invention. Ainsi, la trousse peut notamment comprendre des moyens de mise en œuvre d'une purification préalable des microvésicules, si celle-ci s'avérait utile ou nécessaire. La trousse peut aussi comprendre des tampons utilisables au cours de la mise en œuvre de la méthode selon l'invention, notamment des tampons de suspension des microvésicules, des tampons de lavage, ou des tampons de conservation. Des moyens de détection ou de quantification d'un ou plusieurs marqueurs susceptibles d'être présents sur ou dans les microvésicules, notamment des moyens de détection ou de quantification de marqueurs protéiques ou acides nucléiques peuvent être inclus dans la

trousse. A ce titre, la trousse selon l'invention peut notamment comprendre:

- un support tel que décrit ci-dessus,
- des moyens de détection ou de quantification d'un ou plusieurs marqueurs, et
- facultativement, des tampons utilisables pour la mise en œuvre de la méthode selon l'invention.

Par ailleurs, la trousse peut également comprendre les moyens de détection ou de quantification d'un marqueur de normalisation, notamment un marqueur de normalisation choisi parmi l'annexine-A5 et la beta-actine.

Ainsi, selon un mode particulier de réalisation, la trousse selon l'invention peut comprendre:

- un support tel que décrit ci-dessus,
- des moyens de détection ou de quantification d'un ou plusieurs marqueurs,
- des moyens de détection ou de quantification d'un ou plusieurs marqueurs de normalisation, et
- facultativement, des tampons utilisables pour la mise en œuvre de la méthode selon l'invention.

Selon un autre mode particulier de réalisation, l'invention vise une trousse comprenant:

- un support tel que décrit ci-dessus,
- des moyens de détection ou de quantification de la podocalyxine,
- des moyens de détection ou de quantification d'un marqueur de normalisation, notamment un marqueur de normalisation choisi parmi l'annexine-A5 et la beta-actine, plus particulièrement l'annexine-A5, et
- facultativement, des tampons utilisables pour la mise en œuvre de la méthode selon l'invention.

Selon un autre mode particulier de réalisation, l'invention vise une trousse comprenant:

- un support tel que décrit ci-dessus,
- des moyens de détection ou de quantification de l'alpha-synucléine,
- des moyens de détection ou de quantification d'un marqueur de normalisation, notamment un marqueur de normalisation choisi parmi l'annexine-A5 et la beta-actine, plus particulièrement l'annexine-A5, et
- facultativement, des tampons utilisables pour la mise en œuvre de la méthode selon l'invention.

Selon un autre mode particulier de réalisation, l'invention vise une trousse comprenant:

- un support tel que décrit ci-dessus,
- des moyens de détection ou de quantification de CD41,
- des moyens de détection ou de quantification d'un marqueur de normalisation, notamment un marqueur de normalisation choisi parmi l'annexine-A5 et la beta-actine, plus particulièrement l'annexine-A5, et
- facultativement, des tampons utilisables pour la mise en œuvre de la méthode selon l'invention.

Par "moyens de détection ou de quantification", on entend tout moyen connu de l'homme du métier pour détecter ou quantifier un marqueur. Bien entendu, les moyens employés dépendront de la nature du marqueur, un marqueur protéique pouvant notamment être détecté par des techniques immunologiques (notamment ELISA et Western-blot) et des marqueurs acides nucléiques pouvant être détectés notamment au moyen de techniques d'amplification spécifiques pouvant être qualitatives ou quantitatives (notamment PCR /qPCR ou RT-PCR/RT-qPCR, ou le séquençage). D'autres moyens inclus des tests chromogéniques, selon la nature du biomarqueur à détecter.

Par ailleurs, la trousse selon l'invention peut comprendre une notice fournissant à son utilisateur des directives pour mettre en œuvre la méthode selon l'invention au moyen de la trousse.

L'invention concerne donc également l'utilisation du support selon l'invention pour la capture de microvésicules présentes dans un échantillon de fluide biologique d'un sujet. Les données présentées par les inventeurs montrent qu'il est possible de réaliser une détection de biomarqueurs dans différents types d'échantillons biologiques, notamment dans l'urine ou le sang, plus particulièrement dans le plasma, au moyen de la méthode présentée dans la présente demande. La méthode selon l'invention est donc susceptible de permettre la détection de microvésicules dans un large panel d'échantillons de fluides biologiques. Le fluide biologique peut être, notamment, un échantillon de sang, de sérum, de plasma, de salive, de larmes, d'urine, de liquide lymphatique, de liquide céphalorachidien, ou encore de sperme. Le sujet est un mammifère, notamment un être humain, de tout âge, sexe ou condition. Selon un mode particulier de réalisation, le sujet est un individu chez lequel est suspecté un état pathologique, notamment en raison de tests biologiques ou de consultations médicales réalisées préalablement. Dans un autre mode de réalisation, le sujet n'a pas subi de test biologique ou de consultation médicale préalable.

L'invention vise donc une méthode de capture de microvésicules, comprenant la mise en contact d'un échantillon de fluide biologique susceptible de contenir lesdites vésicules avec un support selon l'invention. Les microvésicules ainsi capturées peuvent ensuite être caractérisées. Selon un mode de réalisation, avant capture avec le support selon l'invention, les microvésicules sont préalablement purifiées ou isolées de l'échantillon de fluide biologique selon des modalités connues de l'homme du métier. Toutefois, les données expérimentales présentées ci-dessous montrent que les microvésicules peuvent être avantageusement capturées directement dans un échantillon de fluide biologique grâce au support selon l'invention. Ainsi, dans un mode particulier de réalisation, les microvésicules sont capturées directement à partir de l'échantillon de fluide biologique, notamment directement à partir d'un échantillon de sang, de sérum, de plasma, de salive, de larmes, d'urine, de liquide lymphatique, de liquide céphalorachidien, ou encore de sperme, plus particulièrement à partir de plasma ou d'urine.

Selon un mode particulier de réalisation, la caractérisation des microvésicules capturées permet le diagnostic d'une pathologie, l'évaluation du risque de développer une pathologie, le pronostic d'une pathologie, le diagnostic différentiel d'une pathologie, le suivi de l'évolution d'une pathologie, ou le suivi de l'efficacité d'un traitement thérapeutique d'une pathologie. Comme nous l'avons rappelé ci-dessus, la composition des microvésicules cellulaires, notamment la composition lipidique et protéique, et le contenu des microvésicules cellulaires (e.g. protéines et matériel génétique, notamment ARN ou ADN mitochondrial), peuvent varier en fonction du type cellulaire dont elles sont originaires, et de l'état de la cellule. Les microvésicules peuvent donc permettre la détection de l'état des cellules dont elles sont originaires, et peuvent donc représenter un outil de choix pour la détection précoce d'un état pathologique. Ainsi, selon un aspect, l'invention concerne l'utilisation d'un support selon l'invention dans une méthode pour le diagnostic, diagnostic différentiel, évaluation du risque, pronostic, suivi de l'évolution ou suivi de l'efficacité d'un traitement thérapeutique de diverses pathologies, notamment thrombotiques, inflammatoires et/ou métaboliques, ou encore de maladies ou accidents cardiovasculaires ou neurovasculaires, ou de maladies comme le diabète, le cancer, la maladie d'Alzheimer, la maladie de Parkinson ou toutes autres pathologies.

L'invention vise donc également une méthode de diagnostic, diagnostic différentiel, d'évaluation du risque, du pronostic, du suivi de l'évolution ou du suivi de l'efficacité d'un traitement thérapeutique de diverses pathologies, notamment thrombotiques, inflammatoires et/ou métaboliques, ou encore de maladies ou accidents cardiovasculaires ou neurovasculaires, ou de maladies comme le diabète, le cancer, la maladie d'Alzheimer, la maladie de Parkinson ou toute autre pathologie On peut ainsi encore citer la néphropathie diabétique, la neuropathie diabétique, la rétinopathie diabétique, la sclérose en plaques, le vasospasme après rupture d'anévrisme, la maladie de Parkinson ou ses dérivés. Selon le but recherché, le résultat de la caractérisation de l'échantillon testé sera comparé au résultat de la caractérisation réalisée sur un échantillon de fluide biologique témoin.

Avantageusement, le fluide biologique témoin est un fluide biologique identique à celui testé, mais provenant d'un sujet considéré comme sain. Alternativement, le fluide biologique témoin provient du même individu que le fluide biologique testé, mais résulte d'un prélèvement antérieur. Un témoin selon cette alternative peut permettre le suivi de l'évolution de la pathologie ou de son traitement.

La caractérisation des microvésicules peut être réalisée par tout moyen connu de l'homme du métier, la nature du marqueur de caractérisation étant prise en compte comme nous l'avons rappelé ci-dessus. Ainsi, dans le cas d'un marqueur protéique, des techniques immunologiques peuvent être employées, mettant en œuvre des anticorps spécifiques dudit marqueur protéique. On peut notamment citer les techniques d'ELISA ou de Western blot. La détection et la caractérisation d'acides nucléiques, notamment de l'ADN mitochondrial ou de l'ARN (notamment ARN messager ou micro-ARN) comprendra l'utilisation de méthodes de détection d'acides nucléiques spécifiques telles que la PCR/qPCR, la RT-PCR/RT-qPCR, le séquençage. D'autres tests, tels que des tests chromogéniques, peuvent être employés selon la nature du biomarqueur à détecter. Avantageusement, une normalisation peut être réalisée sur la base de la quantification d'un marqueur présent sur ou dans les microvésicules. Le marqueur de normalisation peut notamment être choisi parmi l'annexine-A5 et la beta-actine. Par exemple, les données expérimentales présentées ci-dessous montrent que les marqueurs podocalyxine, alpha-synucléine et CD41 peuvent être utilisés pour le diagnostic de pathologies spécifiques. Selon un mode particulier de réalisation, la quantification de ces marqueurs sera réalisée de manière relative par quantification parallèle d'un marqueur de normalisation tel que l'annexine-A5 et la beta-actine. Ainsi, selon un mode particulier de réalisation, la méthode selon l'invention comprend la détection de la quantité d'un biomarqueur présent dans ou sur les microvésicules capturées et la détection de la quantité d'un marqueur de normalisation. Les quantités normalisées sont ensuite comparées pour déterminer un diagnostic, un diagnostic différentiel, réaliser l'évaluation du risque, du pronostic, du suivi de l'évolution ou du suivi de l'efficacité d'un traitement thérapeutique de diverses pathologies.

Selon un second aspect, l'invention vise l'utilisation des biomarqueurs permettant la détection de l'absence ou de la présence d'une pathologie spécifique. Le biomarqueur de caractérisation sera sélectionné en fonction de la pathologie d'intérêt.

Selon un mode de réalisation, l'invention concerne une méthode de diagnostic, notamment de diagnostic précoce, d'une néphropathie chez un sujet, comprenant la détection de la présence ou de l'absence de podocalyxine, ou la mesure du niveau de podocalyxine dans un échantillon de fluide biologique dudit sujet. Selon un mode particulier de

réalisation, la méthode comprend la mesure du ratio podocalyxine/marqueur de normalisation (notamment annexine-A5 ou beta-actine). Plus particulièrement, la méthode selon l'invention peut notamment comprendre la détection de la présence ou de l'absence de microvésicules cellulaires comprenant le marqueur podocalyxine, selon les modalités décrites ci-dessus comprenant l'utilisation d'un support selon l'invention. L'invention vise également l'évaluation du risque de développer une néphropathie, le pronostic d'une néphropathie, le suivi de l'évolution d'une néphropathie, ou le suivi de l'efficacité d'un traitement thérapeutique d'une néphropathie comprenant la détection de la présence ou de l'absence de podocalyxine, ou la mesure du niveau de podocalyxine dans un échantillon de fluide biologique d'un sujet, notamment de podocalyxine comprise dans des microvésicules cellulaires caractérisées selon les modalités exposées ci-dessus. Selon un mode particulier de réalisation, la méthode selon l'invention comprend la quantification du ratio podocalyxine/annexine-A5 ou podocalyxine/beta-actine, plus particulièrement podocalyxine/annexine-A5, pour la détection des complications néphropathiques dans les microvésicules issues d'un échantillon de fluide biologique d'un patient, plus particulièrement un échantillon d'urine. Ce ratio, lorsqu'il est statistiquement supérieur à celui calculé chez un sujet sain, est indicatif d'une néphropathie. Par ailleurs, si ce ratio augmente ou diminue par rapport au ratio calculé par le passé chez le même patient, il témoigne d'une progression ou d'une régression de la néphropatie, respectivement.

Par ailleurs, l'invention concerne une méthode de diagnostic de la maladie de Parkinson chez un sujet, comprenant la détection de la présence ou de l'absence d'alpha-synucléine, ou la mesure du niveau d'alpha-synucléine dans un échantillon de fluide biologique dudit sujet. Selon un mode particulier de réalisation, la méthode comprend la mesure du ratio alpha-synucléine/marqueur de normalisation (notamment annexine-A5 ou beta-actine). Plus particulièrement, la méthode selon l'invention peut notamment comprendre la détection de la présence ou de l'absence de microvésicules cellulaires comprenant le marqueur alpha-synucléine, selon les modalités décrites ci-dessus comprenant l'utilisation d'un support selon l'invention. L'invention vise également l'évaluation du risque de développer la maladie de Parkinson, le pronostic d'une maladie de Parkinson, le suivi de l'évolution d'une maladie de Parkinson, ou le suivi de l'efficacité d'un traitement thérapeutique de la maladie de Parkinson, comprenant la détection de la présence ou de l'absence d'alpha-synucléine, ou la mesure du niveau d'alpha-synucléine dans un échantillon de fluide biologique d'un sujet, notamment d'alpha-synucléine comprise dans des microvésicules cellulaires ou dans les membranes de microvésicules cellulaires caractérisées selon les modalités exposées ci-dessus. Selon un mode particulier de réalisation, la méthode selon l'invention comprend la quantification du ratio alpha-synucléine /annexine-A5 ou alpha-synucléine /beta-actine, plus particulièrement alpha-synucléine /annexine-A5, pour la détection de ces atteintes neurologiques (par exemple, maladie de Parkinson) dans les microvésicules issues d'un échantillon de fluide biologique d'un patient. Ce ratio, lorsqu'il est statistiquement supérieur à celui calculé chez un sujet sain, est indicatif d'une maladie de Parkinson. Par ailleurs, si ce ratio augmente ou diminue par rapport au ratio calculé par le passé chez le même patient, il témoigne d'une progression ou d'une régression de la maladie de Parkinson, respectivement.

L'invention concerne par ailleurs une méthode de diagnostic, notamment de diagnostic précoce, d'une néphropathie diabétique chez un sujet. Selon un mode particulier de réalisation, la méthode selon l'invention comprend la détection de la présence ou de l'absence du marqueur plaquettaire CD41, ou la mesure du niveau de CD41 dans un échantillon de fluide biologique dudit sujet, notamment un échantillon d'urine ou de plasma, plus particulièrement un échantillon de plasma. Selon un mode particulier de réalisation, la méthode comprend la mesure du ratio CD41/marqueur de normalisation (notamment annexine-A5 ou beta-actine). Plus particulièrement, la méthode selon l'invention peut notamment comprendre la détection de la présence ou de l'absence de microvésicules cellulaires comprenant le marqueur CD41, selon les modalités décrites ci-dessus comprenant l'utilisation d'un support selon l'invention. Selon un mode particulier de réalisation, la méthode selon l'invention comprend la quantification du ratio CD41/annexine-A5 ou CD41/beta-actine, plus particulièrement CD41/annexine-A5, pour la détection des complications néphropathiques du diabète dans les microvésicules issues d'un échantillon de fluide biologique d'un patient, notamment un échantillon d'urine ou de plasma. Ce ratio, lorsqu'il est statistiquement supérieur à celui calculé chez un sujet sain, est indicatif d'une néphropathie diabétique. Par ailleurs, si ce ratio augmente ou diminue par rapport au ratio calculé par le passé chez le même patient, il témoigne d'une progression ou d'une régression de la néphropathie diabétique, respectivement.

La détection du stade de néphropathie pourra notamment comprendre la comparaison du résultat de caractérisation obtenu à partir de l'échantillon de fluide biologique test avec le résultat de caractérisation d'un ou plusieurs échantillons témoins caractéristiques de différents stades de la pathologie (notamment provenant de patients diabétiques atteints de néphropathie au stade normoalbuminurique (Normo), microalbuminurique (Micro) ou macroalbuminurique (Macro), ces stades correspondant à la sévérité de la pathologie, le stade Macro étant le plus avancé). Selon un mode particulier de réalisation, la méthode selon l'invention permet ainsi le suivi et le dépistage précoce du diabète et de ses complications potentielles, notamment ses complications rénales ou neuropathiques.

L'invention va maintenant être illustrée de manière non-limitative par les exemples suivants.

## EXEMPLES

Abréviations:

DCM : dichlorométhane
DMSO : diméthylsulfoxide
Eq : équivalent
EtOAc : acétate d'éthyle
MES : 2-(N-morpholino)ethanesulfonic acid
MilliQ : eau purifiée avec un système de purification d'eau Milli-Q
MV : microvésicule
NHS : N-hydroxysuccinimide
NOS : N-oxysuccinimide
Cplx : complexe
PVC : poly(chlorure de vinyle)
PS : phosphatidylsérine
Pht : Phtalimide
MeCN : acétonitrile
XPS : spectrométrie photoélectronique X
EDA : éthylènediamine
HUVEC : Human Umbilical Vein Endothelial Cells

Les complexes utilisés dans cette partie expérimentale ont été synthétisés selon les modalités présentées dans la demande PCT/FR2012/050610, notamment les complexes C1, C2 et C4 de formules:

$NH_2$ · $(ClO_4)_2$ O N Zn O H N N N N Zn

C1;

· $(ClO_4)_2$

C2; et

· $(ClO_4)_2$

C4.

**Exemple 1** : **Fonctionnalisation du support PET et greffage des complexes en surface**

Le polyéthylène téréphtalate (PET) a été choisi pour le greffage covalent des complexes, ce greffage se fait grâce à leur fonction amine primaire réagissant avec les acides carboxyliques présents en surface.

Protocole général :

Tout d'abord, des étapes de fonctionnalisation (**Figure 1**) ont été utilisées pour i) rompre les liaisons esters, ii) augmenter la densité des fonctions COOH, et iii) rendre les carbonyles plus électrophiles grâce à un groupement attracteur et bon groupe partant, le N-hydroxysuccinimide, afin de réagir plus rapidement et avec un meilleur rendement avec les complexes.

1) Le film PET vierge (provenant de Goodfellow) a été pré-découpé en rectangle de 5 cm sur 2 cm afin d'obtenir 10 petits carrés de $1cm^2$. Ce rectangle a été mis à nettoyer dans un tube (falcon) contenant de l'éthanol 96% pendant 1h sous sonication. Le PET a été séché quelques minutes sur papiers Kimtech,
2) Le rectangle de PET vierge nettoyé a été placé dans un autre falcon contenant une solution d'hydroxyde de sodium (NaOH $10^{-3}$ M dans 40 mL MilliQ/MeCN v/v 1/1) pendant 18h à 60°C,
3) Lavage à l'eau MilliQ (3 fois) puis pendant 48h,
4) Oxydation du rectangle de PET dans une solution de permanganate de potassium (2g $KMnO_4$ dans 38.4 mL d'eau MilliQ et 1.6 mL $H_2SO_4$) pendant 1h à 60°C,
5) Lavage acide (HCl 6M) puis lavage à l'eau MilliQ (2 fois) et découpage du rectangle en petits carrés de 1cm sur 1cm,

6) Les carrés ont été mis dans un pilulier avec 2 mL d'une solution de (76.7 mg d'EDC, 23 mg NHS, 39 mg MES dans 2mL d'eau MilliQ pour un carré)

7) Chaque carré est déposé dans un pilulier contenant 2mL de solution de complexe ($10^{-3}$ M dans 0.2 mL de DMSO et 1.8 mL MilliQ) pendant 16h à température ambiante.

8) Rinçage à l'eau MilliQ (3 fois avec sonication) et lavage pendant 48h.

Des échantillons « contrôles » nécessaires pour la caractérisation des matériaux ont été réalisées.

Il s'agit d'échantillons de PET vierge sans hydrolyse ni oxydation et sans activation EDC/NHS. Les étapes 1), 7) et 8) sont réalisées mais sans les étapes 2), 3), 4), 5) et 6).

Caractérisation des matériaux

*Quantification de la densité d'acide carboxylique en surface pour toutes les étapes de fonctionnalisation :*

Pour quantifier la densité de groupes COOH présents en surface du polymère, la méthode utilisée est celle du Toluidine Blue O car ce réactif chimique cationique réagit en surface avec les groupements $COO^-$. En effet, un pH basique (par exemple pH= 10) est nécessaire pour déprotoner les groupements acides carboxyliques afin qu'ils réagissent avec le TBO pour former une liaison non-covalente. Pour résorber le TBO fixé à la surface du matériau, de l'acide acétique est ajouté pour permettre de reprotoner les $COO^-$ et ainsi rompre la liaison ionique [$COO^-$...TBO].

Au préalable, une courbe d'étalonnage est réalisée avec différentes concentrations de TBO. Un report de la densité optique à 630 nm lue pour les échantillons a été fait, et la courbe étalon nous a permis de trouver la concentration de COOH en surface.

Grâce à la courbe étalon, dans le cas du PET vierge (PET livré par le fournisseur et lavé à l'éthanol puis séché), nous obtenons une densité de fonctions COOH de 1,5 pmol/ $mm^2$.

De la même manière, la densité surfacique de fonctions COOH a pu être évaluée sur les surfaces PET aux différentes étapes du traitement. Nous obtenons :

- 28 pmol/$mm^2$ pour le PET hydrolysé
- 210 pmol/$mm^2$ pout le PET oxydé
- 136 pmol/$mm^2$ pour le PET-NHS
- 50 pmol/$mm^2$ pour le PET +C1.

Détermination de pourcentages atomiques en surface du PET aux différentes étapes du traitement par spectrométrie de photoélectrons

Ces analyses ont été réalisées avec le spectromètre « VG ESCALAB 220i-XL ».

La quantification atomique des atomes de Si, S, C, N, O ou Zn a été réalisée par spectrométrie photoélectronique X (XPS), dans le solvant DMSO/$H_2O$ 10/90.

Tableau 1:

| PET NHS activé* | |
|---|---|
| Atome | % Atomique |
| Si | 0,72 ± 0,13 |
| S | 0,28 ± 0,14 |
| C | 70,85 ± 0,13 |
| N | 2,19 ± 0,28 |
| O | 25,98 ± 0,52 |
| *: Groupe N-hydroxysuccinimide sur du PET hydrolysé et oxydé pour augmenter le nombre de COOH et ainsi augmenter le nombre de complexes par unité de surface | |

Tableau 2:

| PET + C1 dans DMSO | |
|---|---|
| Atome | % Atomique |
| Si | 0,34 ± 0,07 |
| S | 0,21 ± 0,04 |
| C | 73,39 ± 0,25 |
| N | 3,76 ± 0,11 |
| O | 20,83 ± 0,30 |
| Zn | 1,48 ± 0,04 |

Les quantifications des pourcentages atomiques permettent de dire que le complexe a bien été immobilisé en surface du PET. En effet, par rapport au PET activé qui ne contient aucun métal, le PET+C1 contient un pourcentage atomique de Zn. De plus, on peut noter une augmentation du pourcentage d'azote attendue théoriquement puisque le complexe contient 6 atomes d'azote au niveau du ligand. Ce résultat est supporté par les rapports tels que COO/C-O qui passe de 0.93 (PET-NHS) à 0.65-0.68 (PET + C1) et NCO/COO qui passe de 0.06 (PET-NHS) à 0.17-0.25 (PET + C1).

Analyse par XPS de supports fonctionnalisés vs non fonctionnalisés, et/ou greffés avec les complexes C1, C2 ou C4

Ces analyses ont été réalisées avec le spectromètre « VG ESCALAB 220i-XL ». 9 échantillons ont été préparés pour l'analyse de photoélectrons X: PET vierge nettoyé (Ech1), PET oxydé (Ech2), PET-NHS ou PET activé (i.e. PET hydrolysé, oxydé puis activé avec EDC/NHS ; Ech3), PET activé + C1 (Ech4), PET activé + C2 (Ech5), PET activé + C4 (Ech6), PET vierge + C1 (Ech7), PET vierge + C2 (Ech8) et PET vierge + C4 (Ech9).

Les spectres hautes résolutions O1s obtenus par XPS montrent que les complexes sont greffés en surface du matériau grâce à l'apparition de pics pour « Zn-O » et « Cu-O » à 530.5-530.7 eV à partir de l'échantillon 4. Au niveau des rapports d'aire Zn-O (ou Cu-O)/C=O, la valeur est de 0.15 pour l'ech4, 0.25 pour l'ech5, 0.11 pour l'ech6 et celle-ci diminue sans l'étape d'activation, 0.08 pour l'ech7, 0.15 pour l'ech8 et 0.14 pour l'ech9. Ces résultats peuvent être expliqués par le fait que sans activation (Ech 7, 8 et 9), les complexes sont adsorbés et non greffés de façon covalente à la surface du support. Ainsi, après plusieurs rinçages, les complexes sont évacués et ne sont presque plus détectés.

Tableau 3:

| % atomique Matériaux | Si | S | C | N | O | Zn ou Cu |
|---|---|---|---|---|---|---|
| **Ech1** | 1,23 ± 1,24 | | 70,57 ± 1,46 | 1,77 ± 0,09 | 26,44 ± 0,28 | |
| **Ech2** | 0,41 ± 0,22 | | 71,85 ± 0,15 | 2,34 ± 0,19 | 25,41 ± 0,2 | |
| **Ech3** | 1,49 ± 0,42 | | 69,55 ± 0,88 | 2,45 ± 0,21 | 26,49 ± 0,35 | |
| **Ech4** | 0,7 ± 0,03 | 0,06 ± 0,03 | 71,92 ± 0,34 | 4,2 ± 0,22 | 22,18 ± 0,52 | 0,93 ± 0,03 |
| **Ech5** | 0,51 ± 0,21 | 0,18 ± 0,03 | 72,27 ± 0,27 | 4,62 ± 0,43 | 21,44 ± 0,51 | 0,97 ± 0,1 |
| **Ech6** | 0,47 ± 0,17 | 0,15 ± 0,02 | 72,17 ± 0,33 | 3,37 ± 0,19 | 23,3 ± 0,22 | 0,53 ± 0,03 |
| **Ech7** | 2,01 ± 0,23 | 0,1 ± 0,02 | 74,4 ± 1,1 | 2,46 ± 0,21 | 20,76 ± 1,26 | 0,27 ± 0,02 |
| **Ech8** | 2,71 ± 0,57 | 0,25 ± 0,04 | 75,6 ± 0,19 | 1,73 ± 0,19 | 19,43 ± 1,18 | 0,27 ± 0,06 |
| **Ech9** | 1,49 ± 0,26 | 0,18 ± 0,08 | 71,56 ± 0,42 | 2,34 ± 0,13 | 23,98 ± 0,68 | 0,43 ± 0,09 |

Quand le greffage covalent a lieu, un pic à 400 eV (spectre haute résolution N1s) correspondant à la liaison amide N-C=O est bien observé par XPS, ce qui signifie que la liaison entre le $NH_2$ du complexe et le carbonyle du matériau est bien effectuée. En outre, quand l'activation EDC/NHS n'est pas effectuée et que les complexes sont mis en contact avec la surface (Ech 7, 8 et 9), un pic différent de celui à 400 eV est observé. Ce pic correspondant au $NH_2$ libre porté par le groupement X du complexe, qui pourrait être corrélé à une adsorption et non à une liaison covalente entre le complexe et le matériau.

<u>Optimisation de la densité des complexes greffés en surface</u>

Afin de n'utiliser que la quantité de complexes nécessaire en surface, de favoriser une meilleure accessibilité des complexes par le phosphatidylsérine présente sur les microvésicules, dans une variante, le support peut être préparé en suivant le protocole général dans lequel les étapes 2 et 3 ne sont pas réalisées ou dans lequel les étapes 2, 3, 4 et 5 ne sont pas réalisées.

En effet, en omettant les étapes d'hydrolyse et/ou d'oxydation du PET, il est possible de moduler la quantité de groupements COOH en surface du PET et donc de moduler la quantité de complexes immobilisés en surface du PET

Dans la variante proposée, les PET vierges sont nettoyés puis fonctionnalisés sans oxydation préalable ou sans oxydation et hydrolyse préalables. Grâce à la variante proposée, seules les fonctions -COOH présentes en surface du support sont activées/fonctionnalisées en vue du greffage de complexes. Avantageusement, le PET n'ayant pas subi d'étape d'oxydation contient moins de groupements acide carboxylique, la densité obtenue de greffage sera donc plus faible (d1)

- 1,5 pmol/ mm2 de COOH pour le PET vierge
- 28 pmol/mm$^2$ de COOH pour le PET hydrolysé
- 210 pmol/mm$^2$ de COOH pout le PET oxydé.

Exemple 2 : Fonctionnalisation du PVC et greffage des complexes

Protocole général :

Le protocole employé va être décrit en référence à la **Figure 2.**

1) Une plaque de PVC de 10 carrés de 1cm$^2$ a été nettoyée par sonication pendant 1h dans 40 mL d'eau MilliQ dans un tube de 50mL. Ensuite, la plaque a été plongée dans une solution comprenant 2 mL d'EDA 97% et 38 mL d'eau MilliQ, pendant 2h à 30°C. Enfin, la plaque de 10 carrés a été rincée 3 fois dans 10mL d'eau MilliQ, une sonication étant réalisée à chaque étape de rinçage.
2) La plaque a été plongée dans une solution comprenant 4 mL de glutaraldéhyde 50% et 30 mL d'eau MilliQ à pH 9,5, pendant 2h à 50°C. Puis la plaque a été rincée 3 fois dans 10mL d'eau MilliQ, une sonication étant réalisée à chaque étape de rinçage.
3) Enfin, le complexe est greffé en plongeant un carré de chaque plaque traitée selon les étapes ci-dessus dans une solution comprenant $10^{-3}$ M de complexe, 0,1 mL de DMSO et 1,9 mL de tampon phosphate citrate (0,5 M acide citrique, 0,5 M phosphate de sodium disodique) à pH4, pendant 16h à température ambiante sous agitation. Puis le carré de PVC a été rincé 3 fois dans 2mL d'eau MilliQ, une sonication étant réalisée à chacune de ces étapes de rinçage. Le carré de PVC traité a ensuite été rincé 3 fois dans 2 mL d'eau MilliQ sur 2 jours.

Caractérisation des surfaces par spectrométrie de photoélectrons (XPS)

Afin de caractériser la surface des matériaux, des analyses par spectrométrie XPS sur le PVC ont été réalisées à chaque étape de leur fonctionnalisation avec le complexe C1. Les matériaux sont schématisés respectivement sur la **Figure 3.** Cinq points de surface, 4 coins et 1 centre, ont été étudiés pour chaque matériau. Ces analyses ont été réalisées avec le spectromètre « VG ESCALAB 220i-XL ».

Résultats

Les spectres et déconvolutions des spectres ainsi que les pourcentages atomiques **(Tableau 3)** des différents éléments présents en surface des matériaux montrent, qu'après fonctionnalisation avec le complexe C1, le Zinc (Zn) est présent à la surface des matériaux et cela à hauteur de 0,25 % pour le PVC. Le Zinc étant le métal constitutif du complexe C1, sa présence nous indique que le complexe C1 est fixé à la surface des matériaux. La fonctionnalisation est donc efficace sur le PVC. De plus, les pourcentages atomiques déterminés en cinq points de surface différents (4 coins + 1 centre) présentent de faibles écarts-types **(Tableau 3)** ce qui démontre l'homogénéité du traitement et la reproductibilité des résultats. De ce fait, nous en déduisons que la fonctionnalisation est homogène sur le PVC.

**Exemple 3 : Greffage du complexe C1 sur plaque** DNA-BIND®

Protocole général

L'immobilisation des complexes sur des plaques commerciales 96 puits DNA-BIND® de Costar a été réalisée. Cette plaque est en polystyrène pré-fonctionnalisé par des fonctions N-oxysuccinimide (NOS). Cette plaque contient 68 x 1014 NOS/cm$^2$. Cette densité de NOS en surface est comparable à la densité de fonctions COOH que nous avions sur le polyéthylène téréphtalate (PET) (8.18 x $10^{15}$ COOH/cm$^2$)

1) On prépare une solution (Vtotal= 2 mL) avec - 1.78g de Complexe - 0.2 mL DMSO - 1.8 mL d'eau MilliQ. 200 μL de cette solution sont déposés dans chaque puits (10 puits). La réaction de greffage est effectuée pendant 16h à température ambiante à l'abri de la lumière.
2) Après ce temps de réaction, la solution est retirée des puits et les puits sont rincés par 3 flushs avec de l'eau MilliQ.

Chaque puits est alors mis à incuber avec une eau MilliQ fraiche et est rincé 3 fois/jour pendant 48h.

Caractérisation des surfaces par spectrométrie de photoélectrons

Afin de caractériser la surface des matériaux, des analyses de spectrométrie XPS sur le « DNA-BIND®» ont été réalisées à chaque étape de leur fonctionnalisation avec le complexe C1. Les matériaux sont schématisés respectivement sur la **Figure 4.** Cinq points de surface, 4 coins et 1 centre, ont été étudiés pour chaque matériau. Ces analyses ont été réalisées avec le spectromètre « VG ESCALAB 220i-XL ».

Résultats

Les spectres et déconvolutions des spectres ainsi que les pourcentages atomiques **(Table 4)** des différents éléments présents en surface des matériaux montrent, qu'après fonctionnalisation avec le complexe C1, le Zinc (Zn) est présent à la surface des matériaux et cela à hauteur de 0,41 % pour le « DNA-BIND®». Le Zinc étant le métal constitutif du complexe C1, sa présence nous indique que le complexe 1 est immobilisé en surface des matériaux. La fonctionnalisation est donc efficace sur le « DNA-BIND®». De plus, les pourcentages atomiques déterminés en cinq points de surface différents (4 coins + 1 centre) présentent de faibles écarts-types **(Tableau 4)** ce qui démontre l'homogénéité du traitement et la reproductibilité des résultats. De ce fait, nous en déduisons que la fonctionnalisation est homogène sur les deux matériaux : PVC et « DNA-BIND®».

Tableau 4:

| Matériaux / % Atomique | PVC vierge | PVC EDA | PVCGlu. | PVC + C1 | DNA-BIND® vierge | DNA-BIND® + C1 |
|---|---|---|---|---|---|---|
| C | 74,06 +/- 0,03 | 73,74 +/- 0,51 | 74,90 +/- 0,34 | 78,87 +/- 1,40 | 96,52 +/- 0,26 | 91,59 +/- 2,25 |
| N | 0,51 +/- 0,23 | 0,81 +/- 0,29 | 0,67 +/- 0,07 | 2,08 +/- 0,20 | 0,78 +/- 0,09 | 1,73 +/- 0,09 |
| O | 6,58 +/- 0,12 | 6,27 +/- 0,49 | 8,43 +/- 0,45 | 8,69 +/- 0,21 | 2,60 +/- 0,26 | 5,69 +/- 1,81 |
| Cl | 18,64 +/- 0,25 | 18,89 +/- 1,07 | 15,7 +/- 0,25 | 9,46 +/- 1,15 | / | / |
| Zn | / | / | / | **0,25 +/- 0,05** | / | **0,41 + /- 0,03** |
| Si | 0,18 +/- 0,03 | 0,18 +/- 0,09 | 0,18 +/- 0,05 | 0,50 +/- 0,12 | 0,16 +/- 0,09 | 0,16 +/- 0,05 |
| S | 0,10 | 0,18 +/- 0,16 | 0,07 | 0,12 +/- 0,04 | / | 0,28 +/- 0,21 |
| Na | / | / | / | / | / | 0,44 +/- 0,28 |
| Sn | / | 0,15 | 0,17 | 0,11 | / | / |

**Tableau 4.** Pourcentages atomiques des éléments présents en surface des matériaux PVC et « DNA-BIND®» obtenus par XPS après chaque étape de leur fonctionnalisation avec le complexe 1 sur cinq points de surface (4 coins + 1 centre) ainsi que les écarts-types correspondant.

**Exemple 4** : **Recherche de marqueurs portés par les microvésicules suite à leur capture par le kit polystyrène** DNA-BIND® **greffé avec le complexe C1**

Matériels et méthodes

a/ Prélèvements de fluides biologiques de patients diabétiques

Les prélèvements d'urines et de plasma de patients diabétiques ont été effectués dans le service de diabétologie du Pr Vincent Rigalleau. Tous les patients ont signé un consentement de façon libre et éclairé en accord avec la Déclaration d'Helsinski et approuvé par le comité d'éthique. Le tableau clinique, dont le taux d'albuminurie dans les urines, est établi pour chaque patient diabétique. Trois groupes de patients se distinguent : les patients Normoalbuminuriques ou Normo (Stade 1 des complications de la pathologie), les Microalbuminuriques ou Micro (Stade 2) et les Macroalbuminuriques ou Macro (Stade 3). Des prélèvements d'urines de donneurs sains, sans complication néphropathique connue, sont également collectés. Tous les prélèvements sont conservés à -80°C jusqu'à utilisation.

b/ Modèle cellulaire Human Umbilical Vein Endothelial Cells HUVECs

Les cellules endothéliales, Human Umbilical Vein Endothelial Cells (HUVECs) (Promocell, C-12208) sont cultivées en milieu Endothelial Cell Growth Medium 2 Kit (Promocell, C-22111) composé d'un milieu basal et d'un mélange de facteurs de croissance sans antibiotiques.

c/ Isolement de microvésicules modèles à partir de culture cellulaire (Human Umbilical Vein Endothelial Cells ou HUVECs)

Pour l'activation de la production de microvésicules modèles, les cellules HUVECs sont rincées 2 fois avec du tampon PBS et sont stimulées pendant 24h dans du milieu de culture avec 100 ng/mL de TNF-alpha (Peprotech, ref 300-01A). Les milieux de culture des cellules sont récupérés après 24h de stimulation et centrifugés en tube de 50 mL à 12000g pendant 2 min à 4°C afin d'éliminer les cellules, débris cellulaires et corps apoptotiques. Les surnageants sont transférés dans des tubes propres et centrifugés à 20000g pendant 90 min à 4°C afin de sédimenter les microvésicules. Le culot de microvésicules modèles obtenu est lavé par resuspension dans 1,5 mL de tampon PBS froid, transféré dans un tube de 1,5 mL puis centrifugé à 20000g pendant 90 min à 4°C. Cette étape de lavage est renouvellée une fois. Le culot de microvésicules modèles est ensuite resuspendu selon la taille du culot dans du PBS froid (100 à 500 μL). La quantité de protéines correspondantes est estimée par absorbance à 280 nm à l'aide d'un spectrophotomètre, nanodrop. L'échantillon de microvésicules modèles est conservé à -80°C jusqu'à utilisation.

d/ Purification de microvésicules à partir de prélèvements d'urines humaines

Les urines de donneurs sains et de patients sont décongelées et la purification des microvésicules est obtenu par centrifugations différentielles : une première centrifugation est réalisée à 12000g pendant 2 min à 4°C afin d'éliminer les cellules, débris cellulaires et corps apoptotiques. Le surnageant est centrifugé à 20000g pendant 90 min à 4°C afin de sédimenter les microvésicules. Le culot de microvésicules obtenu est lavé par resuspension dans 1,5 mL de tampon PBS froid, transféré dans un tube de 1,5 mL puis centrifugé à 20000g pendant 90 min à 4°C. Cette étape de lavage est renouvelée une fois. L'échantillon est conservé à -80°C jusqu'à utilisation.

e/ Purification de microvésicules à partir de prélèvements de sang humain

Le sang collecté dans un tube sodium citrate est soumis à une première centrifugation à 1500 g. La phase supérieure correspondant au plasma est prélevée soigneusement et est centrifugée à 12000 g pendant 2 minutes afin d'éliminer les plaquettes. Le surnageant correspondant au platelet-free plasma, PFP, est centrifugé ensuite à 20000 g à 4°C afin de sédimenter les microvésicules. Le culot de microvésicules obtenu est lavé par resuspension dans 1,5 mL de tampon PBS froid, transféré dans un tube de 1,5 mL puis centrifugé à 20000g pendant 90 min à 4°C. Cette étape de lavage est renouvellée une fois. L'échantillon est conservé à -80°C jusqu'à utilisation.

f/ Détection de biomarqueurs protéiques

Méthode Enzymatique : Les microvésicules modèles produites par activations des cellules HUVECs présentent à leur surface des activateurs du plasminogène qui activent le plasminogène (présent à la surface des microvésicules) en plasmine; l'activité de cette plasmine peut être détectée à l'aide d'un substrat chromogène, le méthylmalonyl-hydroxypropyl-arginyl-paranitroanilide, CBS0065. Le chromophore est clivé en présence de plasmine en un produit de couleur jaune dont l'absorbance peut être mesurée à l'aide d'un spectrophotomètre à la longueur d'onde de 450 nm. Les microvésicules resuspendues dans du tampon PBS après isolement à partir d'HUVECs sont déposées dans les puits de la

plaque de 96 puits en polystyrène (DNA-BIND® Costar) greffés avec le complexe métallique dinucléaire I appelé complexe I. Une quantité totale de 2,5 μg de microvésicules (dosage réalisé par la technique de nanodrop A280 nm) dans 25 μL est incubée 1h à température ambiante. Après trois rinçages, l'activité enzymatique de chaque puits est estimée. Certains puits ne sont pas rincés et représentent le contrôle 100 % de l'activité enzymatique. Dans un premier temps, une gamme étalon est obtenue par dilution en cascade avec les concentrations suivantes en activateur du plasminogène en UI/mL : 0 ; 0,00078125 ; 0,0015625 ; 0,003125 ; 0,00625 ; 0,0125 ; 0,025 et 0,05. Dans un deuxième temps, une solution de plasminogène à 4 μM et une solution du substrat chromogène CBS0065 à 3 mM sont préparées puis mélangées volume à volume. Un volume de 25 μL de ce mélange est ajouté dans chaque puits. La plaque est recouverte d'un film autocollant et placée dans le spectrophotomètre à 37°C pendant 18h pour la lecture de l'absorbance simultanément à 405 nm et à 450 nm dans chaque puits. On réalise ainsi une cinétique ce qui nous permet de calculer la vitesse moyenne d'apparition du produit reflétant l'activité enzymatique de chaque puits, elle est exprimée en mOD/min.

g/ Western blot

La première étape consiste en la lyse des microvésicules à l'aide du tampon RIPA + inhibiteurs de protéases et de phosphatases (990 μL de tampon RIPA + 10 μL du cocktail des inhibiteurs de protéases et de phosphatases (100X)). Ajouter un volume de tampon RIPA+inhibiteurs en fonction de la taille du culot de microvésicules obtenu lors de la dernière étape d'isolement avant ajout de PBS (volume de RIPA environ 4x supérieur au volume du culot). Le culot est resuspendu par des allers-retours à la pipette et laissé dans la glace 15 minutes. Puis le lysat est centrifugé à 10000xg pendant 10 minutes à 4°C afin d'éliminer les débris. Le surnageant est transféré dans un tube propre. La quantité de protéines présente dans le lysat de microvésicules est mesurée par la technique de BiCinchoninic acid Assay (BCA). Les protéines sont ensuite dénaturées à l'aide du tampon Laemmli 4X+ agent réducteur (DTT, dithiothreitol). Le lysat dénaturé peut être conservé à -80°C avant utilisation. Les lysats dénaturés de microvésicules sont déposés sur gel d'acrylamide en conditions dénaturantes SDS-PAGE à gradient de concentration 4-12%. La migration est réalisée sous tension constante de 120 V pendant environ 1h30 (jusqu'à que le front de migration atteigne l'extrémité du bas du gel). Le transfert du gel est effectué sur une membrane de PVDF. Suite à la saturation de la membrane 1h à température ambiante avec une solution de lait 5% dans du tampon TBS-tween 0.1%, les 3 anticorps primaires dilués dans la solution de lait 5% en TBS-tween 0.1% sont incubés pendant une nuit à 4°C. Les dilutions utilisées sont les suivantes : anticorps dirigé contre la podocalyxine (Santacruz, sc-23904) 1/2000 ou contre CD41 (Novus, MAB 7616) au 1/1000 ; plus anticorps contre la beta-actine (SIGMA, A1978-100UL) 1/10000 ; plus anticorps contre l'annexine-A5 (SIGMA, A8604-100UL) 1/2000. Après deux lavages en tampon TBS-tween 0.1%, les anticorps secondaires couplés à l'enzyme HorseRadish Peroxidase, HRP, et à la dilution 1/5000, sont incubés 1h à température ambiante dans la solution lait 5% en TBS-tween 0.1%. La révélation par chemiluminescence est réalisée après deux lavages en tampon TBS-tween0.1%. Le signal est capté par une caméra CCD puis l'intensité de celui-ci est analysée par le logiciel gratuit ImageJ.

h/ Analyse statistique

Les statistiques des résultats de western blot pour la validation du biomarqueur dans les microvésicules issues d'urines de patients diabétiques ont été analysées avec le logiciel graphpad Prism version 7.

i/ ELISA

Les microvésicules resuspendues dans du tampon PBS après isolement à partir d'urines ou de plasma de patients ou d'individus sains sont déposées dans les puits de la plaque de 96 puits en polystyrène (DNA-BIND® Costar) greffés avec le complexe métallique dinucléaire I appelé complexe I. Une incubation d'une nuit à 4°C sous agitation horizontale douce est observée. Suite à deux lavages en PBS, la solution de saturation composée de 5% de protéine « Bovine Serum Albumine» (BSA) est déposée dans tous les puits de la plaque et incubée 2h à température ambiante sous agitation douce. Puis, les anticorps primaires dirigés soit contre la podocalyxine (santacruz, sc-23904) au 1/500, soit contre le CD41, marqueur des plaquettes (Novus, MAB 7616) au 1/500 sont mis en contact avec les microvésicules captées dans les puits pendant 2h à température ambiante sous agitation horizontale douce. Suite à deux lavages, les anticorps secondaires couplés à l'enzyme HRP, à la dilution 1/5000, sont incubés pendant 1h à température ambiante sous agitation horizontale douce. Après six lavages, 100 μL révélateur chromogène TMB (3,3',5,5'-tétraméthylbenzidine) sont déposés dans les puits pour une durée de 30 minutes à température ambiante sous agitation horizontale douce. La réaction est stoppée par l'ajout de 100 μL d'acide sulfurique et l'absorbance de chaque puit est mesurée à l'aide d'un spectrophotomètre à 450nm.

Résultats

a/ Identification de marqueurs protéiques portes par des microvesicules

*Validation du biomarqueur podocalyxine dans les microvésicules issues d'urines de patients diabétiques selon le stade de complication néphropathique par la technique de western blot.*

La podocalyxine est une protéine transmembranaire exprimée par les cellules podocytes, cellules glomérulaires présentes uniquement dans le rein, elle est décrite dans la littérature comme marqueur de lésion cellulaire des podocytes.

La protéine beta-actine est une protéine du cytosquelette exprimée dans toutes les cellules ; cette expression universelle et stable, fait qu'elle est souvent utilisée en western blot comme protéine de référence lorsque l'on doit quantifier le taux d'une protéine dont l'expression peut varier en fonction de la pathologie.

La protéine annexine-A5 est localisée proche de la membrane plasmique dans toutes les cellules ; tout comme la beta-actine, elle est aussi utilisée comme protéine de référence en western blot. De ce fait, l'analyse de l'expression de la protéine podocalyxine par western blot dans les microvésicules issues d'urines de donneurs sains ou de patients diabétiques sera donc plutôt exprimée sous forme de ratio : ratio podocalyxine/beta-actine et ratio podocalyxine/annexine-A5.

La **Figure 5** représente le ratio podocalyxine/beta-actine. Chaque point représente le ratio obtenu après analyse par western blot de l'échantillon de microvésicules d'urines provenant d'un donneur sain ou d'un patient diabétique. On observe que les donneurs sains expriment un ratio faible de podocalyxine/beta-actine en moyenne de 0,70, cela correspond au taux basal de production naturelle de microvésicules issues de podocytes dans des conditions non pathologiques. Ce ratio augmente progressivement selon les stades de néphropathie liée au diabète. On remarque ainsi que le ratio podocalyxine/beta-actine passe en moyenne à 1,09 chez les patients normoalbuminuriques, puis à 1,70 chez les patients microalbuminuriques et enfin à 3,91 chez les patients macroalbuminuriques. L'analyse statistique ANOVA, avec le test de Tukey's de comparaison inter-groupe, par le logiciel GraphPad Prism, révèle une différence significative entre les groupes : Sain versus Macro avec une p-value : $p<0.001$, Normo versus Macro avec $p<0.01$ et Micro versus Macro avec $p<0.05$.

La **Figure 6** représente le ratio podocalyxine/annexine-A5. Chaque point représente le ratio obtenu après analyse par western blot de l'échantillon de microvésicules d'urines provenant d'un donneur sain ou d'un patient diabétique. On observe comme précédemment pour le ratio podocalyxine/beta-actine que les donneurs sains expriment un ratio faible de podocalyxine/annexine-A5 en moyenne de 0,50, cela correspond au taux basal de production naturelle de microvésicules issues de podocytes dans des conditions non pathologiques. A nouveau, le ratio augmente progressivement selon les stades de néphropathie liée au diabète. On remarque ainsi que le ratio podocalyxine/annexine-A5 passe en moyenne à 0,93 chez les patients normoalbuminuriques, puis à 2,15 chez les patients microalbuminuriques et enfin à 4,33 chez les patients macroalbuminuriques. L'analyse statistique ANOVA, avec le test de Tukey's de comparaison inter-groupe, par le logiciel GraphPad Prism, révèle une différence significative entre les groupes : Sain *versus* Normo avec $p<0.05$, Sain *versus* Macro avec $p<0.0001$, Normo *versus* Macro avec $p<0.0001$ et Micro *versus* Macro avec $p<0.01$.

En conclusion, on peut envisager que le ratio podocalyxine/annexine-A5 soit utilisé dans le cadre d'une trousse de détection des complications néphropathiques dans les microvésicules issues des urines des patients. Ce ratio, lorsqu'il augmente, témoigne d'une lésion cellulaire des podocytes.

*Détection du marqueur plaquettaire, CD41, dans les microvésicules issues de plasma de patients diabétiques selon le stade de complication néphropathique par la technique de western blot*

Le but de nos expériences dans cette partie est de montrer que nous sommes capables de détecter un marqueur dans les microvésicules purifiées par centrifugation issues de plasma humain. Pour cela, nous avons mis au point la détection du CD41, protéine localisée à la surface des membranes des plaquettes et ce par la technique du western blot. Les microvésicules provenant du bourgeonnement des membranes des plaquettes expriment CD41. Ainsi, nous avons analysé le ratio de l'expression du CD41/annexine-A5 dans les microvésicules issues de plasma d'un patient diabétique Normoalbuminurique et d'un patient diabétique Macroalbuminurique (ci-dessous Figure 7). Nous pouvons observer la détection spécifique du marqueur plaquettaire CD41 dans les extraits protéiques de microvésicules issues de plasma par la technique de western blot. Le ratio CD41/annexine-A5 varie selon les individus. Cette expérience montre qu'il est possible de quantifier dans le plasma un marqueur porté par les microvésicules circulantes.

*Conclusion*

Nous avons montré que nous pouvons quantifier des biomarqueurs protéiques dans les microvésicules isolées à partir de différents fluides biologiques : urine et plasma.

De plus, le ratio podocalyxine / annexine-A5 pourrait être utilisé dans une trousse de diagnostic de détection de complication néphropatique.

b/ Validation de la surface de polystyrène DNA-BIND® greffé avec le complexe C1

*Capture de microvésicules modèles et détection enzymatique de biomarqueurs portés par ces microvésicules.*

Les microvésicules sont porteuses de matériel biologique telles que les enzymes, des protéines à activité biologique. Nous nous sommes servis de la technique enzymatique décrite dans le brevet PCT/FR2012/050610 pour valider le greffage du complexe C1 sur la surface de polystyrène DNA-BIND®. La capture des microvésicules modèles par le matériau est réalisée avec une quantité de 2,5 μg de microvésicules totales (équivalent protéique) dans 25 μL pendant 1h à température ambiante. L'activité enzymatique présente dans chaque condition expérimentale, est analysée par la cinétique enzymatique. C'est-à-dire que l'on va suivre la vitesse d'apparition d'un produit chromogène, le CBS0065, catalysé par l'enzyme pendant 18h. Les conditions expérimentales sont les suivantes :

*Condition non rincée* 100%: c'est la condition contrôle, les microvésicules modèles sont laissées dans les puits pendant 1h et l'activité enzymatique est estimée directement sans rinçage des puits. L'activité enzymatique détectée est l'activité maximale de l'extrait de microvésicules modèles. Cette condition représente donc le témoin positif, le 100%.

*Condition rincée sans complexe* : les microvésicules modèles sont incubées 1h dans les puits de la plaque non greffée avec le complexe C1 puis rincées trois fois et l'activité enzymatique est analysée. Cette condition représente le témoin négatif, l'activité enzymatique obtenue témoigne d'une interaction non spécifique des microvésicules modèles.

*Condition rincée avec complexe* : les microvésicules modèles sont incubées 1h dans les puits de la plaque greffée avec le complexe C1 puis rincées trois fois et l'activité enzymatique est analysée. Cette condition représente la capture des microvésicules par le complexe, l'activité enzymatique obtenue témoigne d'une interaction spécifique des microvésicules modèles.

La **Figure 8** représente les moyennes des Vi (moyenne de la vitesse initiale calculée) obtenues pour chaque condition décrite ci-dessus. On observe une moyenne de la Vi de 0,226 mOD/min dans la condition contrôle des microvésicules non rincées, ce qui représente la moyenne de Vi maximale obtenue pour 2,5μg de microvésicules, c'est donc le 100%. Dans les puits où les microvésicules modèles sont incubées sur support non greffé avec le complexe métallique, on mesure une moyenne de la vitesse initiale de 0,026 mOD/min. Le taux d'interaction non spécifique est de : 0,026/0,226 = 0.115 soit 11.5%. Dans les puits où les microvésicules sont incubées sur support greffé avec le complexe métallique C1 puis rincées, on mesure une moyenne de la vitesse initiale de 0,152 mOD/min. Le taux de capture spécifique est donc de : 0,152/0,226 = 0,672 soit 67,2%. En conclusion, cette expérience montre (1) que la surface de polystyrène DNA-BIND® greffé avec le complexe C1 permet une capture des microvésicules de modèle cellulaire HUVECs et (2) que la surface de polystyrène DNA-BIND® greffé avec le complexe C1 permet une quantification enzymatique portée par les microvésicules modèles. De plus, nous pouvons évaluer une capture spécifique de 67,2% des microvésicules.

*Capture des microvésicules isolées à partir d'urines humaines et détection par ELISA d'un marqueur podocytaire porté par ces microvésicules*

Nous avons testé la présence de la podocalyxine et sa détection par la méthode immunologique ELISA pour valider la capture spécifique des microvésicules isolées à partir d'urines et démontrer la capacité de quantification d'un biomarqueur après capture sur notre matériau. Pour cela, nous avons incubé les microvésicules isolées par centrifugation à partir d'urines une nuit à 4°C sous agitation douce sur la plaque de polystyrène DNA-BIND® Costar-96 puits greffée entièrement avec le complexe C1. Nous avons déposé une quantité maximale de microvésicules équivalente à 20 μg de protéines dosées par spectrophotométrie (soit 100 ng/μL dans les 200μL de réaction finale). Après plusieurs lavages, nous avons détecté la présence de la protéine podocalyxine à l'aide d'un anticorps spécifiquement dirigé contre elle. Un anticorps secondaire couplé à l'enzyme HorseRadish Peroxydase est ajouté afin d'amplifier et de détecter un signal par spectrophotométrie à 450 nm en présence d'un substrat à propriété chromogène le TMB. Les résultats sont rapportés dans la figure 9. Nous pouvons ainsi remarquer un signal spécifique dans la condition : Microvésicules capturées par le matériau + anticorps primaire contre la podocalyxine + anticorps secondaire. Un signal équivalent au bruit de fond est observé dans toutes les autres conditions correspondantes aux différents contrôles. Nous pouvons conclure que les microvésicules isolées à partir d'urine humaine ont été captées spécifiquement et que la podocalyxine à la surface des microvésicules est détectée spécifiquement.

Afin de démontrer que la détection par la technique d'ELISA du biomarqueur de la protéine podocalyxine dans les microvésicules issues d'urines humaines après capture sur matériaux peut être considérée comme semi-quantitative, nous avons réalisé une gamme de dépôts des microvésicules comprises entre 0,5 μg et 20 μg soit des concentrations comprises entre 2,5 ng/μL et 100 ng/μL dans 200μL de réaction finale. Les résultats des mesures d'absorbances en fonction des différentes quantités de microvésicules sont représentés dans la **figure 10.** Nous pouvons observer une relation linéaire entre la concentration de microvésicules déposées et captées par le matériau et le signal détecté pour la

protéine podocalyxine par la technique d'ELISA.

Ainsi, le matériau (complexe C1 greffé sur la plaque de polystyrène DNA-BIND® de Costar) permet non seulement la capture spécifique des microvésicules isolées à partir d'urines mais permet aussi de quantifier la présence de la podocalyxine, par la technique d'ELISA avec un seuil de détection de 2,5 ng/µL de microvésicules déposées.

*Capture des microvésicules isolées à partir de plasma humain et détection par ELISA du marqueur plaquettaire CD41 porté par ces microvésicules circulantes*

Nous avons voulu montrer que le matériau (complexe C1 greffé sur la plaque de polystyrène DNA-BIND® de Costar) est aussi capable de capter les microvésicules circulantes (i-e : isolées du plasma humain) isolées préalablement par centrifugation et qu'il est possible de quantifier par la technique d'ELISA la présence d'une protéine sur les microvésicules captées. Pour cela, nous avons choisi la protéine, CD41, présente à la surface des plaquettes et donc retrouvée à la surface des microvésicules issues de leur bourgeonnement membranaire. Les microvésicules circulantes isolées préalablement par centrifugation à partir de plasma de patients diabétiques normoalbuminuriques ont été déposées dans la plaque de polystyrène fonctionnalisée avec le complexe C1 et incubées une nuit à 4°C sous agitation douce. Nous avons déposé par puits une quantité de microvésicules isolées à partir de 1,5 mL de plasma. Après plusieurs lavages, nous avons détecté la présence de la protéine CD41 à l'aide d'un anticorps spécifiquement dirigé contre elle. Un anticorps secondaire couplé à l'enzyme HorseRadish Peroxydase est ajouté afin d'amplifier et de détecter un signal par spectrophotométrie à 450 nm en présence d'un substrat à propriété chromogène le TMB. Les résultats sont rapportés dans la **figure 11.** Nous observons un signal spécifique dans les puits où les microvésicules de plasma de patients (P1 et P2) ont été déposées. Ces résultats indiquent non seulement la capture spécifique des microvésicules circulantes mais aussi la possibilité de quantifier par la technique d'ELISA la présence d'une protéine à leur surface, ici la protéine plaquettaire CD41.

*Capture des microvésicules contenues dans le plasma humain et détection du biomarqueur protéique plaquettaire, CD41, par ELISA*

Enfin, nous avons voulu montrer que le matériau (complexe C1 greffé sur la plaque de polystyrène DNA-BIND® de Costar) est capable de capter les microvésicules non isolées. L'échantillon est donc ici non pas des microvésicules préalablement isolées par centrifugation, mais directement du plasma humain contenant des microvésicules. Nous avons cherché à quantifier par la technique d'ELISA la présence de la protéine CD41 portée par les microvésicules de l'échantillon de plasma. Pour cela, nous avons incubé 300 µL de plasma de patients diabétiques normoalbuminuriques une nuit à 4°C sous agitation douce sur la plaque de polystyrène DNA-BIND® greffée avec le complexe C1. Après plusieurs lavages, nous avons détecté la présence de la protéine CD41 à l'aide d'un anticorps spécifiquement dirigé contre elle. Un anticorps secondaire couplé à l'enzyme HorseRadish Peroxydase est ajouté afin d'amplifier et de détecter un signal par spectrophotométrie à 450 nm en présence d'un substrat à propriété chromogène le TMB. Les résultats sont rapportés dans la **Figure 12.** Bien que les taux soient faibles, nous observons un signal pour la détection de CD41 dans les puits où les plasmas de patients (P3, P4 et P5) ont été déposés dans les puits greffés avec le complexe C1. Ces résultats indiquent non seulement la capture spécifique des microvésicules (non purifiées préalablement) présentes dans le plasma mais aussi la possibilité de quantifier par la technique d'ELISA la présence d'une protéine à leur surface, ici la protéine plaquettaire CD41.

**Exemple 5** : **Diagnostic précoce d'une néphropathie diabétique par capture sur support solide de microvésicules cellulaires**

Matériel et méthodes

*Prélèvement de fluides biologiques humains* : Tous les prélèvements ont été conservés à -80°C jusqu'à leur utilisation.

Prélèvements urinaires humains de sujets sains : Les prélèvements urinaires humains ont été réalisés à l'institut de Chimie et Biologie des Membranes et des Nano-objets (CBMN) à Pessac sur des sujets sains en journée dans des pots à urine classiques sans ajout d'additifs. Le début de la miction n'a pas été récupéré et les prélèvements ont été conservés à -80°C jusqu'à leur utilisation.

Prélèvements urinaires humains de patients diabétiques : Les donneurs sont des patients diabétiques atteints de néphropathie au stade normoalbuminurique (Normo), microalbuminurique (Micro) ou macroalbuminurique (Macro). Ces stades correspondent à la sévérité de la pathologie, le stade Macro étant le plus avancé. Les prélèvements urinaires ont été effectués en journée dans des pots à urine classiques sans ajout d'additifs. De plus, le début de la miction n'a pas été récupéré.

Purification des microvésicules

Microvésicules issues d'un modèle cellulaire : Les cellules endothéliales humaines de veine de cordon ombilicale, HUVEC (Promocell ; réf. C-12208), ont été cultivées dans du milieu complet (Promocell ; Endothelial Cell Growth Medium 2 Kit ; réf. C-22111) selon les recommandations du fournisseur. Entre les passages 3 et 6, les cellules ont été rincées deux fois en PBS 1X, puis activées avec une solution de TNF-alpha (Peprotech ; réf. 300-01A) préparée à 100 ng/mL dans du milieu complet pendant 24h à 37°C afin de produire des microvésicules. Ensuite, le surnageant contenant les microvésicules a été transféré dans un tube 50 mL. Dans un premier temps, les débris cellulaires et corps apoptotiques ont été culotés par centrifugation à 12 000 g 2 min à 4°C (SIGMA ; centrifugeuse thermostatée 3-18KHS) afin de les éliminer. Dans un deuxième temps, le surnageant contenant les microvésicules a été transféré dans un nouveau tube de 50 mL et les microvésicules ont été culotées par centrifugation à 20 000 g 1h30 à 4°C. Le surnageant a été éliminé et le culot rincé avec 1 mL de solution tampon (HEPES 10 mM ; NaCl 0,15 M ; pH 7,4). La solution de microvésicules a été transférée dans un nouveau microtube de 1,5 mL et centrifugée à 20 000 g 1h30 à 4°C. Le surnageant a été éliminé et le culot de microvésicules de nouveau rincé puis centrifugé à 20 000 g 1h 30 à 4°C. Ce dernier culot de microvésicules a enfin été resuspendu en solution tampon (HEPES 10 mM ; NaCl 0,15 M ; pH 7,4) et cette solution stockée à -80°C jusqu'à son utilisation.

Microvésicules issues de prélèvement urinaire humain : Afin d'isoler les microvésicules, les urines ont été décongelées 1 nuit à 4°C et 40 mL ont été centrifugés à 1 500 g 15 min à 4°C. Le surnageant fut transféré dans un nouveau tube et centrifugé à 12 000 g 2 min à 4°C pour éliminer les débris cellulaires et corps apoptotiques. Le surnageant fut ensuite transféré dans un nouveau tube et centrifugé à 20 000 g 1h30 à 4°C afin de culoter les microvésicules. Le surnageant a été retiré et le culot rincé dans 1 mL de tampon (HEPES 10 mM ; NaCl 0,15 M ; pH 7,4). La solution de microvésicules a été transférée dans un microtube de 1,5 mL puis centrifugée à 20 000 g 1h30 à 4°C. Le surnageant a été éliminé et le culot de microvésicules de nouveau rincé puis centrifugé à 20 000 g 1h 30 à 4°C. Ce dernier culot de microvésicules a enfin été resuspendu en solution tampon (HEPES 10 mM ; NaCl 0,15 M ; pH 7,4) et cette solution stockée à -80°C jusqu'à son utilisation.

Observation en CRYOMEB

Capture des microvésicules : Une solution tampon (HEPES 10 mM ; NaCl 0,15 M ; pH 7,4) contenant des microvésicules (issues de l'activation cellulaire des cellules HUVEC ou issues de prélèvement urinaire humain de sujet sain) a été incubée 1 nuit à température ambiante, à raison de 300 μL par matériau, puis éliminée par 10 rinçages successifs avec la solution tampon. Les matériaux utilisés sont le carré d'1 $cm^2$ de PVC + C1 et le puits de plaque « DNA-BIND® » + C1.

Observation en CRYOMEB : Les échantillons ont été observés avec un Microscope Electronique à Balayage (MEB) à effet de champ « Quanta 250 FEG FEI » équipé d'un module cryo « Quorum PP3000T ». Les échantillons ont d'abord été collés sur un support grâce à un mélange Tissue-Tek®/carbone, puis rapidement refroidis dans l'azote pâteux. Ils ont ensuite été insérés dans la première chambre du microscope pour procéder à une sublimation durant 20 min à -95°C puis dans la deuxième chambre pour procéder à la métallisation par pulvérisation de Platine sous Argon à 10 mA pendant 1 min. Enfin, les échantillons métallisés ont été introduit dans la chambre d'observation pour l'acquisition des images.

Capture des microvésicules sur support DNA-BIND®

Dosage des protéines : Les protéines ont été dosées par NanoDrop (ND1000 ; Thermo Fisher SCIENTIFIC) afin d'estimer la quantité de microvésicules présentes dans chaque solution.

Capture des microvésicules : Le matériau utilisé, également appelé « trousse» dans la présente demande, est la plaque 96 puits « DNA-BIND® » (COSTAR ; réf. 2525) fonctionnalisée avec le complexe C1. L'expérience est divisée en deux parties : une partie capture des microvésicules par la trousse et une partie microvésicules contrôles hors de la plaque ainsi non captées par la trousse. La partie « contrôle » nous informe sur la composition initiale des microvésicules sans la capture. Une solution tampon (HEPES 10 mM ; NaCl 0,15 M ; pH 7,4) contenant des microvésicules est alors séparée en deux parties égales : une partie pour la capture et une partie pour les contrôles.

*Partie capture des microvésicules* : La solution tampon (HEPES 10 mM ; NaCl 0,15 M ; pH 7,4) contenant des microvésicules a été incubée sur les matériaux 1h à 37°C ou 1 nuit à 4°C à raison de 50 μL par puits. La plaque a été recouverte avec un film afin d'éviter toute évaporation. La solution tampon a ensuite été éliminée et les puits rincés trois fois 10 min avec la même formule de tampon à raison de 200 μL/puits afin d'isoler les microvésicules captées du reste des objets présents en solution et d'éliminer ceux qui seraient adsorbés sur le matériau de manière non spécifique. Les microvésicules ont ensuite été lysées dans 30 μL de tampon de lyse (99% dans tampon RIPA - 1% de cocktail inhibiteurs de protéases) par puits durant 30 min dans la glace afin de récupérer toutes les protéines des microvésicules individuellement. Après homogénéisation, la solution a été transférée dans un microtube de 1,5 mL. Les débris

membranaires ont été culotés par centrifugation à 10 000 g pendant 10 min à 4°C afin de les éliminer. Le surnageant contenant les protéines a été récupéré dans un nouveau microtube.

*Partie microvésicules contrôles* : La solution tampon (HEPES 10 mM ; NaCl 0,15 M ; pH 7,4) contenant des microvésicules a été incubée en microtube 1,5 mL 1h heure à 37°C ou 1 nuit à 4°C à raison de 50 μL par microtube. Après homogénéisation de la solution, les microvésicules ont été culotées par centrifugation à 20 000 g pendant 1h30 à 4°C. Le surnageant a été retiré par aspiration. Les microvésicules ont ensuite été resuspendues et lysées dans 30 μL de tampon de lyse par microtube durant 30 min dans la glace afin de récupérer toutes les protéines des microvésicules individuellement. Après homogénéisation de la solution, les débris membranaires ont été culotés par centrifugation à 10 000 g pendant 10 min à 4°C afin de les éliminer. Le surnageant contenant les protéines a été récupéré dans un nouveau microtube.

*Analyse par Western blot :*

Chaque échantillon (extrait protéique) a été préparé dans un microtube avec du tampon de charge (Bolt™ LDS Sample Buffer 4X + Bolt™ Sample Reducing Agent 10X) à raison de 19,5 μL de solution de protéines pour 30 μL d'échantillon final selon les proportions indiquées par le fournisseur. Les échantillons ont été vortexés et rapidement centrifugés avec une centrifugeuse de paillasse avant d'être dénaturés à 95°C pendant 5 min. La totalité de l'échantillon (30 μL) a été déposée sur gel d'acrylamide en conditions dénaturantes. Un marqueur de taille a toujours été ajouté comme référence. Les échantillons ont migré 10 min à 50 V puis 1h30 à 120V. Les protéines présentes dans le gel ont été transférées sur membrane PVDF par transfert semi-liquide pendant 15 min à 1,3 A constants. Les sites aspécifiques de la membrane ont été saturés dans un bain de tampon lait (5 % de lait écrémé en tampon Tris Buffer Saline (TBS) - Tween 0,1%) pendant 1h à température ambiante sous agitation. Après deux rinçages de 10 min en tampon TBS - Tween 0,1% sous agitation, la membrane a été incubée dans une solution d'anticorps primaires, préparée en tampon lait, une nuit à 4°C sous agitation. Les anticorps (Ac) utilisés sont l'Ac anti-podocalyxine (Santacruz, réf. sc-23904 ; dilution 1 / 2 000), l'Ac anti-beta-actine (Sigma, réf. A1978 ; dilution 1 / 5 000) et l'Ac anti-annexine-A5 (Sigma, réf. A8604 ; dilution 1 / 2 000). Après deux rinçages de 10 min en tampon TBS - Tween 0,1% sous agitation, la membrane a été incubée dans une solution d'Ac secondaires, préparée en tampon lait, 1h à RT sous agitation. Le signal a été détecté par un imageur de gel (GeneGnome, SYNGENE) grâce à une réaction de chimiluminescence après incubation de la membrane avec un révélateur en Pico 5 min (ThermoFisher SCIENTIFIC, réf. 34580) ou en Femto 2 min (ThermoFisher SCIENTIFIC, réf. 34094) en fonction de l'intensité du signal. L'acquisition des images a été effectuée avec le logiciel GeneSys. Une analyse, à la fois qualitative et quantitative, a été réalisée sur Image J. L'aspect qualitatif a été apprécié par la présence de bandes, au poids moléculaire spécifique de chaque marqueur d'intérêt, ainsi que par l'intensité de leur signal. L'aspect quantitatif a été déterminé par le calcul d'un ratio : l'intensité du signal du marqueur pathologique, la podocalyxine, par rapport à l'intensité du signal du marqueur de référence des microvésicules, l'annexine-A5. Ce ratio permet de déterminer la quantité de marqueur pathologique relative au nombre de microvésicules.

**Résultats**

Analyse CRYOMEB

Les images de la capture des microvésicules par le PVC + C1 sont représentées sur la **Figure 13** (microvésicules issues de l'activation cellulaire des cellules HUVEC) et celles de la capture par le « DNA-BIND® » + C1 sont représentées sur les **Figure 14** (microvésicules issues de l'activation cellulaire des cellules HUVEC) et **Figure 15** (microvésicules issues de prélèvement urinaire humain de sujet sain). Ces images montrent la présence de nombreux objets sphériques de 40 nm à 100 nm de diamètre pour le PVC + C1 **(Figure 13)** et en moyenne de 150 nm sur le « DNA-BIND® » + C1 **(Figure 14** et **Figure 15)** et donc de microvésicules phosphatidylsérine-positives capturées par les différents matériaux testés. Les plaques 96-puits fonctionnalisées par C1 captent les microvésicules issues d'un modèle cellulaire (HUVEC).

Capture des microvésicules et révélation par Western blot

Dans un premier temps, la capture des microvésicules par la trousse a été démontrée par la détection du marqueur de référence, l'annexine-A5 porté par des microvésicules modèles, après incubation sur la trousse à deux doses différentes. Les résultats sont représentés sur la **Figure 16.** La présence d'une bande significative au poids moléculaire correspondant à l'annexine-A5 après capture témoigne du recrutement spécifique et efficace des microvésicules. De plus, la variation de l'intensité du signal en fonction de la quantité de microvésicules déposées indique une dose-réponse également spécifique. Dans un deuxième temps, la capture des microvésicules par la trousse a été démontrée par la détection de l'annexine-A5 sur des microvésicules issues de prélèvements urinaires humains de sujets sains. Les résultats sont représentés sur la **Figure 17.** La présence d'une bande significative au poids moléculaire de

l'annexine-A5 après incubation sur la trousse témoigne d'une capture spécifique des microvésicules urinaires. Après validation de la capture, nous avons testé la détection d'un biomarqueur pathologique sur les microvésicules captées par la trousse. Le biomarqueur étudié est la podocalyxine, témoin de néphropathie au niveau podocytaire. Les microvésicules utilisées sont issues de prélèvements urinaires d'un sujet sain et d'un patient diabétique atteint de néphropathie au stade microalbuminurique (Micro). Les résultats sont représentés sur la **Figure 18** et la **Figure 19.** Les données relatives aux donneurs sont indiquées dans le tableau 5.

Tableau 5: Données cliniques relatives aux donneurs représentés sur la Figure 18 et Figure 19.

| **Type de donneur** | **Patient diabétique** | **Sujet sain** |
|---|---|---|
| **Numéro d'identification du donneur** | Micro 1 | Sain 1 |
| **Stade albuminurie** | Micro | / |
| **Dosage microalbuminurie (mg/mmol)** | 8,8 | / |
| **Dosage microalbuminurie (mg/jour)** | 58,6 | / |
| **Date de naissance** | 05/01/1981 | / |
| **Age (ans)** | 36 | 25 |
| **Sexe** | Masculin | Masculin |
| **Type diabète** | DT2 | / |
| **Année découverte** | 2009 | / |
| **Durée diabète (années)** | 8 | / |
| **Poids (kg)** | 115,1 | 68 |
| **Taille (cm)** | 171 | 178 |
| **IMC (Indice de Masse Corporelle, kg/m$^2$)** | 39,3 | 21 |
| **TA (Tension Arterielle) (mmHg)** | 140/95 | / |
| **Macroangiopathie** | Oui | / |
| **HB1Ac (Hémoglobine glyquée, %)** | 7 | / |
| **GAJ (Glycémine A jeun)** | 0,88 | / |
| **Creatinine (µmol/L)** | 134 | / |
| **DFG (Débit de Filtration Glomérulaire, mL/min)** | 67 | / |
| **Hématurie** | Non* | / |
| **Leucocyturie** | Non* | / |
| **Statine** | Oui* | / |
| **Bloqueur SRAA (Système Rénine Angiotensine Aldostérone)** | Oui* | / |
| **Diurétique** | Non* | / |
| **Aldactone** | Non* | / |
| **Gradation du risque d'ulcération du pied** | 0 | / |
| *(Oui : le patient prend le traitement ; Non : le patient ne prend pas le traitement.) | | |

La présence des bandes caractéristiques de la podocalyxine et de l'annexine-A5 après capture sur la trousse, mise en évidence sur la **Figure 18,** montre que le Western Blot permet de détecter un marqueur pathologique après capture sur la trousse. De plus, la quantification du signal, représentée par la **Figure 19,** montre que le ratio podocalyxine/annexine-A5, qui semble être révélateur de la pathologie, est du même ordre de grandeur sur les microvésicules sans la trousse (représentées en noir) et sur celles qui sont captées par la trousse (représentées en gris). Ce résultat montre que la population de microvésicules captées est représentative de la population initialement présente dans le fluide biologique ce qui permet d'effectuer un diagnostic pertinent. Enfin, nous avons comparé le ratio podocalyxine/annexine-A5 des microvésicules issues de prélèvements urinaires de sujets sains et de patients diabétiques atteints de

néphropathie aux différents stades de la pathologie (Normo, Micro, Macro) sans capture et après capture par la trousse. Les résultats sont représentés sur la **Figure 20** et les informations cliniques relatives aux donneurs sont indiquées dans le tableau 6.

Tableau 6: Données cliniques relatives aux donneurs représentés sur la Figure 20.

| Type de donneur | Sujet sain | Sujet sain | Patient diabétique | Patient diabétique | Patient diabétique | Patient diabétique | Patient diabétique |
|---|---|---|---|---|---|---|---|
| Numéro d'identification du donneur | Sain 2 | Sain 3 | Normo 1 | Normo 2 | Micro 2 | Macro 1 | Macro 2 |
| Stade albuminurie | / | / | Normo | Normo | Micro | Macro | Macro |
| Dosage microalbuminurie (mg/mmol) | / | / | 0 | 0 | 8,7 | 260,4 | 368 |
| Dosage microalbuminurie (mg/jour) | / | / | 0 | 0 | 77,2 | 2864 | 3275 |
| Date de naissance | / | / | 18/05/1949 | 03/12/1975 | 28/03/1982 | 22/01/1982 | 17/03/1946 |
| Age (ans) | 27 | 25 | 67 | 41 | 75 | 35 | 71 |
| Sexe | Féminin | Féminin | Féminin | Masculin | Masculin | Masculin | Féminin |
| Type diabète | / | / | DT1 | DT1 | DT2 | DT1 | DT1 |
| Année découverte | / | / | 2014 | 2006 | 2007 | 1993 | 2007 |
| Durée diabète (années) | / | / | 3 | 11 | 10 | 26 | 11 |
| Poids (kg) | 55 | 53 | 42 | 89 | 121 | 62,2 | 96 |
| Taille (cm) | 160 | 161 | 164 | 175 | 168 | 180 | / |
| IMC (Indice de Masse Corporelle, kg/m²) | 21 | 20 | 15,6 | 29 | 42,8 | 19 | 34,1 |
| TA (Tension Arterielle) (mmHg) | / | / | 137/82 | 110/60 | 120/60 | 137/84 | 155/83 |
| Macroangiopathie | / | / | Non | Non | Non | Non | Non |
| HB1Ac (Hémoglobine glyquée, %) | / | / | 11,5 | 9 | 8 | 7,3 | 8,1 |
| GAJ (Glycémine A jeun) | / | / | 2,34 | 1,4 | 1,51 | 1,66 | 1,62 |
| Creatinine (µmol/L) | / | / | 70 | 72 | 75 | 207 | 44 |
| DFG (Débit de Filtration Glomérulaire, mL/min) | / | / | 77 | 110 | 85 | 35 | 98 |
| Hématurie | / | / | N | / | Oui | / | / |
| Leucocyturie | / | / | N | / | Non | / | / |
| Statine | / | / | O | N | Oui | Oui | Oui |
| Bloqueur SRAA (Système Rénine Angiotensine Aldostérone) | / | / | N | N | Oui | Oui | Oui |
| Diurétique | / | / | N | N | Oui | Oui | Oui |
| Aldactone | / | / | N | N | Oui | Non | Oui |
| Gradation du risque d'ulcération du pied | / | / | 0 | / | 1 | / | / |
| *(Oui : le patient prend le traitement ; Non : le patient ne prend pas le traitement.) | | | | | | | |

Le graphique de la Figure 20 montre que les ratios calculés à partir des microvésicules après capture sur la trousse (en gris) sont représentatifs des ratios calculés à partir des microvésicules sans capture (en noir) quel que soit le stade de la pathologie (Normo, Micro ou Macro). La technique de détection Western Blot permet donc de valider le fait que la trousse peut être utilisée pour effectuer un diagnostic précoce et apprécier la sévérité d'une pathologie.

## Revendications

**1.** Support solide comprenant, un composé de formule (I) ou (II) suivante qui est lié de manière covalente au support de manière directe ou indirecte:

(I)

(II)

dans laquelle

- $M^{+i}$ représente un ion métallique et i est 1, 2 ou 3 ;
- L représente un ligand échangeable ;
- X représente un groupe $-(CH_2)_m-NH_2$, ou un groupe $-CH_2-NHC(O)-R-NH_2$ dans lequel R est un groupe alkyle en $C_2-C_{10}$, en particulier en $C_5-C_{10}$, substitué ou non substitué, linéaire ou ramifié ;
- m = 1 à 12 ;
- n = 1, 2 ou 3 ; et
- Y représente H ou $(CH_2)_p-NH_2$, où p = 0 à 12,

dans lequel, dans le cas d'une liaison covalente indirecte, ledit composé de formula (I) ou (II) est lié de manière covalente à une fonction réactive apportée à la surface du support par un agent de pré-fonctionnalisation, qui a également été lié de manière covalente au support.

**2.** Support solide selon la revendication 1, ledit support solide étant **caractérisé en ce qu'**il s'agit d'un support solide en poly(chlorure de vinyle) (PVC), en poly(téréphtalate d'éthylène) (PET) ou en polystyrène (PS).

**3.** Support solide selon la revendication 1 ou 2, **caractérisé en ce qu'**il s'agit d'un support solide en PET pré-

fonctionnalisé au moyen de fonctions NHS, en PVC fonctionnalisé au moyen de glutaraldéhyde, ou en PS fonctionnalisé au moyen de fonctions N-oxysuccinimide.

4. Support solide selon l'une quelconque des revendications 1 à 3, **caractérisé en ce que** M est choisi parmi Zn, Cu, Mn, Co, Ni et Fe, en particulier parmi Zn et Cu, M étant plus particulièrement Zn.

5. Support solide selon l'une quelconque des revendications 1 à 4, **caractérisé en ce que** :

- X représente $-(CH_2)_m-NH_2$, m= 1, n=1 et Y représente H ; ou
- X représente $-(CH_2)_m-NH_2$, m= 1, n=2 et Y représente H ; ou
- X représente -CH2-NHC(O)-R-$NH_2$, R représente $C_5H_{10}$, n=1 ou 2 et Y représente H.

6. Support solide selon l'une quelconque des revendications 1 à 5, **caractérisé en ce que** le composé lié de manière covalente au support de manière directe ou indirecte est un composé de formule (I), notamment choisi parmi:

H
N
NH₂
O
(ClO₄)₂
Zn
Zn
N
N
N
N
N
N
O
H

$\cdot (ClO_4)_2$

et

$\cdot (ClO_4)_2$

**7.** Méthode de capture de microvésicules présentes dans un échantillon de fluide biologique d'un sujet, comprenant la mise en contact dudit échantillon avec un support selon l'une quelconque des revendications 1 à 6.

**8.** Méthode de caractérisation de microvésicules présentes dans un échantillon de fluide biologique d'un sujet, comprenant une étape de capture desdites microvésicules après mise en contact dudit échantillon avec un support selon l'une quelconque des revendications 1 à 6, et une étape de caractérisation des microvésicules capturées.

**9.** Méthode pour le diagnostic d'une pathologie, comprenant une étape de capture des microvésicules présentes dans un échantillon de fluide biologique d'un sujet sur un support selon l'une quelconque des revendications 1 à 6, et la détection de la présence ou l'absence d'un marqueur associé à ladite pathologie, ou la mesure du niveau dudit marqueur caractéristique de ladite pathologie.

**10.** Méthode selon la revendication 9, comprenant :

- la détection de la présence ou l'absence de podocalyxine, ou la mesure du niveau de podocalyxine à partir des microvésicules capturées sur le support, pour le diagnostic d'une néphropathie ; ou
- la détection de la présence ou l'absence d'alpha-synucléine, ou la mesure du niveau d'alpha-synucléine à partir des microvésicules capturées sur le support, pour le diagnostic de la maladie de Parkinson ; ou
- la détection de la présence ou l'absence de CD41, ou la mesure du niveau de CD41 à partir des microvésicules capturées sur le support, pour le diagnostic d'une néphropathie diabétique.

**11.** Trousse comprenant:

- un support selon l'une quelconque des revendications 1 à 6; et
- des moyens de détection ou de quantification d'un marqueur associé à une pathologie.

**12.** Trousse selon la revendication 11, le marqueur étant associé à une pathologie étant la podocalyxine, l'alpha-synucléine ou CD41.

**13.** Trousse selon la revendication 11 ou 12, comprenant en outre des moyens de détection ou de quantification d'un marqueur de normalisation.

**14.** Trousse selon la revendication 13, marqueur de normalisation étant l'annexine-A5 ou la beta-actine.

**Patentansprüche**

**1.** Fester Träger, umfassend eine Verbindung der folgenden Formel (1) oder (II), die direkt oder indirekt kovalent an den Träger gebunden ist:

(I)

(II)

,

wobei

- $M^{+i}$ ein Metallion darstellt und i 1, 2 oder 3 ist;

- L einen austauschbaren Liganden darstellt;
- X eine $-(CH_2)_m-NH_2$-Gruppe oder eine $-CH_2-NHC(O)-R-NH_2$-Gruppe darstellt, wobei R eine $C_2$-$C_{10}$-Alkylgruppe, insbesondere eine $C_5$-$C_{10}$-Alkylgruppe, substituiert oder unsubstituiert, linear oder verzweigt ist;
- m = 1 bis 12;
- n = 1, 2 oder 3; und
- Y H oder $(CH_2)_p-NH_2$ darstellt, wobei p = 0 bis 12 ist,

wobei im Falle einer indirekten kovalenten Bindung diese Verbindung der Formel (I) oder (II) kovalent an eine reaktive Funktion gebunden ist, die durch ein Präfunktionalisierungsmittel an die Oberfläche des Trägers gebunden wurde, die ebenfalls kovalent an den Träger gebunden ist

**2.** Der feste Träger nach Anspruch 1, wobei dieser Träger **dadurch gekennzeichnet ist, dass** es sich um einen festen Träger aus Polyvinylchlorid (PVC), aus Polyethylenterephthalat (PET) oder aus Polystyrol (PS) handelt.

**3.** Der feste Träger nach Anspruch 1 oder 2, **dadurch gekennzeichnet, dass** es sich um einen festen Träger aus aus mit mindestens NHS-Funktionalitäten vorfunktionalisiertem PET, aus mit mindestens Glutaraldehyd funktionalisiertem PVC oder aus mit mindestens N-Oxysuccinimid-Funktionalitäten funktionalisiertem PS besteht.

**4.** Der feste Träger nach einem der Ansprüche 1 bis 3, **dadurch gekennzeichnet, dass** M ausgewählt ist aus Zn, Cu, Mn, Co, Ni und Fe, insbesondere aus Zn und Cu, wobei M im Speziellen Zn ist.

**5.** Der feste Träger nach einem der Ansprüche 1 bis 4, **dadurch gekennzeichnet, dass**:

- X für $-(CH_2)_m-NH_2$ steht, m = 1, n = 1 und Y für H steht; oder
- X für $-(CH_2)_m-NH_2$ steht, m = 1, n = 2 und Y für H steht; oder
- X für $-CH_2-NHC(O)-R-NH_2$ steht, R für $C_5H_{10}$ steht, n = 1 oder 2 und Y für H steht.

**6.** Ein fester Träger nach einem der Ansprüche 1 bis 5, **dadurch gekennzeichnet, dass** die direkt oder indirekt kovalent an den Träger gebundene Verbindung eine Verbindung der Formel (I) ist, insbesondere ausgewählt aus:

NH2
N
O
Zn
Zn
N
N
N
N
N
O
H

und

7. Verfahren zum Einfangen von Mikrovesikeln in einer biologischen Flüssigkeitsprobe eines Patienten, umfassend das Inkontaktbringen dieser Probe mit einem Träger gemäß einem der Ansprüche 1 bis 6.

8. Verfahren zur Charakterisierung von Mikrovesikeln in einer biologischen Flüssigkeitsprobe eines Patienten, um-

fassend einen Schritt des Einfangens dieser Mikrovesikel nach dem Inkontaktbringen dieser Probe mit einem Träger gemäß einem der Ansprüche 1 bis 6 und das anschließende Charakterisieren der eingefangenen Mikrovesikel.

**9.** Verfahren zur Diagnose einer Erkrankung, umfassend einen Schritt des Einfangens von Mikrovesikeln, die in einer biologischen Flüssigkeitsprobe eines Patienten vorliegen, auf einem Träger gemäß einem der Ansprüche 1 bis 6 und die anschließende Detektion des Vorhandenseins oder Fehlens eines mit der Erkrankung assoziierten Markers bzw. das Messen der Konzentration dieses für die Erkrankung charakteristischen Markers.

**10.** Verfahren nach Anspruch 9, umfassend:

- die Detektion des Vorhandenseins oder Fehlens von Podocalyxin oder die Messung des Podocalyxin-Gehalts in auf dem Trägermaterial gebundenen Mikrovesikeln zur Diagnose von Nephropathie; oder
- die Detektion des Vorhandenseins oder Fehlens von Alpha-Synuclein oder die Messung des Alpha-Synuclein-Gehalts in auf dem Trägermaterial gebundenen Mikrovesikeln zur Diagnose der Parkinson-Krankheit; oder
- die Detektion des Vorhandenseins oder Fehlens von CD41 oder die Messung des CD41-Gehalts in auf dem Trägermaterial gebundenen Mikrovesikeln zur Diagnose von diabetischer Nephropathie.

**11.** Kit, umfassend:

- einen Träger nach einem der Ansprüche 1 bis 6; und
- Mittel zur Detektion oder zur Quantifizierung eines mit einer Pathologie assoziierten Markers.

**12.** Kit nach Anspruch 11, wobei der mit einer Pathologie assoziierte Marker Podocalyxin, Alpha-Synuclein oder CD41 ist.

**13.** Kit nach Anspruch 11 oder 12, ferner umfassend Mittel zur Detektion oder zur Quantifizierung eines Normalisierungsmarkers.

**14.** Kit nach Anspruch 13, wobei der Normalisierungsmarker Annexin-A5 oder Beta-Actin ist.

## Claims

**1.** A solid support comprising, a compound of the following formula (I) or (II) which is bound covalently to the support directly or indirectly:

(I)

(II)

in which

- $M^{+i}$ represents a metal ion and i is 1, 2 or 3;
- L represents an exchangeable ligand;
- X represents a group $-(CH_2)_m-NH_2$, or a group $-CH_2-NHC(O)-R-NH_2$ in which R is a $C_2$-$C_{10}$, in particular $C_5$-$C_{10}$, alkyl group, substituted or unsubstituted, linear or branched;
- m = 1 to 12;
- n = 1, 2 or 3; and
- Y represents H or $(CH_2)_p-NH_2$, where p = 0 to 12,

wherein in the case of an indirect covalent bond, the compound of formula (I) or (II) is bound covalently to a reactive function supplied to the surface of the support by a prefunctionalization agent, which has also been bound covalently to the support.

2. The solid support as claimed in claim 1, said solid support being **characterized in that** it is a solid support made of poly(vinyl chloride) (PVC), poly(ethylene terephthalate) (PET) or polystyrene (PS).

3. The solid support as claimed in claim 1 or 2, **characterized in that** it is a solid support of PET prefunctionalized by means of NHS functions, of PVC functionalized by means of glutaraldehyde, or of PS functionalized by means of N-oxysuccinimide functions.

4. The solid support as claimed in any one of claims 1 to 3, **characterized in that** M is selected from Zn, Cu, Mn, Co, Ni and Fe, in particular from Zn and Cu, M being more particularly Zn.

5. The solid support as claimed in any one of claims 1 to 4, **characterized in that**:

- X represents $-(CH_2)_m-NH_2$, m= 1, n=1 and Y represents H; or
- X represents $-(CH_2)_m-NH_2$, m= 1, n=2 and Y represents H; or
- X represents $-CH_2-NHC(O)-R-NH_2$, R represents $C_5H_{10}$, n=1 or 2 and Y represents H.

6. The solid support as claimed in any one of claims 1 to 5, **characterized in that** the compound covalently bound to the support, directly or indirectly, is a compound of formula (I), in particular selected from:

· (ClO4)2
H
N
NH2
O
N
O
N
N
Zn
Zn
N
O
H
N
N

and

7. A method for capturing microvesicles present in a sample of biological fluid from a subject, comprising bringing said sample into contact with a support as claimed in any one of claims 1 to 6.

8. A method for characterization of microvesicles present in a sample of biological fluid from a subject, comprising a step of capture of said microvesicles after bringing said sample into contact with a support as claimed in any one of claims 1 to 6, and a step of characterization of the captured microvesicles.

9. A method for the diagnosis of a disease, comprising a step of capturing the microvesicles present in a sample of biological fluid from a subject on a support as claimed in any one of claims 1 to 6, and detection of the presence or absence of a marker associated with said disease, or measurement of the level of said marker characteristic of said disease.

10. The method as claimed in claim 9, comprising:

- detecting the presence or absence of podocalyxin, or measurement of the level of podocalyxin from the microvesicles captured on the support, for the diagnosis of a nephropathy; or
- detecting the presence or absence of alpha-synuclein, or measurement of the level of alpha-synuclein from the microvesicles captured on the support, for the diagnosis of Parkinson's disease; or
- detecting the presence or absence of CD41, or measurement of the level of CD41 from the microvesicles captured on the support, for the diagnosis of a diabetic nephropathy.

11. A kit comprising:

- a support as claimed in any one of claims 1 to 6; and
- means for detecting or quantifying a marker associated with a disease.

12. The kit as claimed in claim 11, the marker associated with a disease is podocalyxin, alpha-synuclein or CD41.

13. The kit as claimed in claim 11 or 12, further comprising means for detecting or quantifying a normalization marker.

14. The kit as claimed in claim 13, normalization marker is annexin-A5 or beta-actin.

FIGURE 1

PET vierge
1) NaOH, $H_2O$, $CH_3CN$ pendant 18h à 60°C
2) $KMnO_4$ dans $H_2SO_4$ (1.2N) pendant 1h à 60°C
PET oxydé
1) MES (0.1M), EDC (0.2M), NHS (0.1M) dans MilliQ pendant 17h à 4°C
2) $10^{-3}$ M complex dans dmso/eau pendant 16h à $T_{amb}$
Complexes greffés sur PET
COOH

**FIGURE 2**

Lavage du PVC

Plaque de 10 carrés de 1cm²
sont nettoyées dans 40 mL de
MilliQ (tube 50 mL).
Sonication pendant 1h

1) Réaction avec EDA:

2 mL EDA
38 mL MilliQ
30°C, 2h

RINCAGES: 3 x 10 mL MilliQ sonicatior

2) Réaction avec Glutaraldéhyde:

4 mL Glutaraldéhyde 50%
30 mL Eau MilliQ

50°C, 2h

RINCAGES: 3 x 10 mL MilliQ sonicat°

3) Greffage du complexe

$10^{-3}$ M de complexe
0.1 mL de DMSO
1.9 mL de tampon phosphate Citrate pH4
16h sous agitation à TA

RINCAGES: 3 x 2 mL MilliQ sonicat°
2jours 3 x 2 mL MilliQ

FIGURE 3

A – PVC vierge
Support : PVC = poly(chlorure de vinyle), $(C_2H_3Cl)_n$
B – PVC EDA
C – PVC Glu.
Gluta = glutaraldéhyde $(C_5H_8O_2)$
D – PVC + C1
Complexe 1

FIGURE 4

A – « DNA-BIND® » vierge
NOS (N-oxy-succinimide)
Support = PS (Polystyrene) $(C_8H_8)_n$
R1 = inconnu (donnée confidentielle du fournisseur)
B – « DNA-BIND® » + C1
Complexe 1

FIGURE 5

***
**
*
Ratio PODOCALYXINE/BETA-ACTINE en unite arbitraire (U.A)
10
8
6
4
2
0
0.70
1.09
1.70
3.91
Sain
Normo
Micro
Macro
n=14
n=15
n=18
n=7

FIGURE 6

****
****
*
**
Ratio PODOCALYXINE/ANNEXINE - A5 en unité arbitraire (U.A)
10
8
6
4
2
0
0.50
0.93
2.151
4.33
Sain
Normo
Micro
Macro
n=1
n=1
n=23
n=10

FIGURE 7

A
Normo
Macro
CD41
BETA-ACTINE
ANNEXINE-A5
B
CD41/ANNEXIN A5 Ratio
1,6
1,4
1,2
1
0,8
0,6
0,4
0,2
0
0,44
1,57
NORMO
MACRO

FIGURE 8

0,300
0,250
0,200
0,150
0,100
0,050
0,000
Vi (mOD/min)
0,226
0,026
0,152
Condition non rincée (100 %)
Condition sans complexe
Condition avec complexe

FIGURE 9

0,35
0,3
0,25
0,2
0,15
0,1
0,05
0
Absorbance à 450nm
(unité arbitraire)
TMB + + + + +
Anticorps primaire - + - + +
Anticorps secondaire - - + + +
Microvésicules + - - - +

FIGURE 10

0,4
0,35
0,3
0,25
0,2
0,15
0,1
0,05
0
Absorbance à 450nm
(unité arbitraire)
y = 0,003x + 0,0511
R² = 0,9828
0 10 20 30 40 50 60 70 80 90 100
Concentration de microvésicules
(ng/µL équivalent protéique)

FIGURE 11

Détection immunologique de CD41 dans les microvésicules isolées à partir de plasma humain
Absorbance à 450nm (unité arbitraire)
0,12
0,1
0,08
0,06
0,04
0,02
0
Ctl
P1
P2

**FIGURE 12**

Détection de CD41 après capture des microvésicules de plasma humain
Absorbance à 450nm (unité arbitraire)
0,05
0,04
0,03
0,02
0,01
0
P3
P4
P5

FIGURE 13

A
B
C
MV

FIGURE 14

A
MV
B
MV

FIGURE 15

A
B
MV
C
MV

FIGURE 16

MVs HUVECs
5 µg de protéines
MVs HUVECs
10 µg de protéines
Ctrl
Trousse
Ctrl
Trousse
Annexine-A5
MVs du
sujet sain
1
MVs du
sujet sain
2
Ctrl
Trousse
Ctrl
Trousse
Annexine-A5

FIGURE 17

FIGURE 18

MVs du sujet sain
MVs du patient (Micro)
Ctrl
Trousse
Ctrl
Trousse
PODOCALYXINE
Annexine-A5

## FIGURE 19

1,4
1,2
1
0,8
0,6
0,4
0,2
0
Ratio
PODOCALYXINE / Annexine-A5
1,13
1,03
0,71
0,31
Sujet sain
Patient (Micro)
Ctrl : MVs sans le kit
Kit

1,4
1,2
1,0
0,8
0,6
0,4
0,2
0,0
Ratio
PODOCALYXINE / Annexine-A5
1,13
1,03
0,71
0,31
Sujet sain
Patient (Micro)
Ctrl : MVs sans la trousse
Trousse

# FIGURE 20

Contrôle : MVs sans la trousse
Trousse
2,0
1,8
1,6
1,4
1,2
1,0
0,8
0,6
0,4
0,2
0,0
Sain 1
Sain 2
Normo 1
Normo 2
Micro 1
Macro 1
Macro 2
Donneurs

SAINS
NORMO
MACRO
Contrôle : MVs sans le kit
Kit
Ratio
2,00
1,80
1,60
1,40
1,20
1,00
0,80
0,60
0,40
0,20
0,00
Sain 2
Sain 3
Normo 1
Normo 2
Micro 2
Macro 1
Macro 2
Donneurs

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2012050610 W
- ES 2340131
- CN 103333878
- CN 107754014


### Littérature non-brevet citée dans la description


- **HARA et al.** *Diabetologia*, 2012, vol. 55, 2913-2919
- **STUENDL et al.** *Brain*, 2015, vol. 139 (2), 481-494
- **RODRIGUES et al.** *Journal of Diabetology & Metabolic Syndrome*, 2018, vol. 10 (1), A60
- **MILLER et al.** *Neuroscience Letters*, 2005, vol. 374, 189-191
- **STEELE et al.** *Blood*, 2010, vol. 116 (22), 4569-4577
- **WANG et al.** *Nanoscale*, 2015, vol. 7, 3548